# EUROPEAN PATENT APPLICATION

(11) **EP 2 495 240 A1**
(43) Date of publication of application: **05.09.2012**
(21) Application number: 10826322.9
(22) Date of filing: 26.10.2010
(51) Int. Cl.: C07D 307/91, C09K 11/06, H01L 51/50

(54) **FLUORANTHENE COMPOUND AND ORGANIC ELECTROLUMINESCENT ELEMENT USING SAME**

(30) Priority: 26.10.2009 JP 2009245865
(71) Applicant: Idemitsu Kosan Co., Ltd., Chiyoda-ku Tokyo 100-8321 (JP)
(72) Inventor: MIZUKI, Yumiko, Sodegaura-shi Chiba 299-0293 (JP); FUNAHASHI, Masakazu, Sodegaura-shi Chiba 299-0293 (JP)
(74) Representative: Gille Hrabal
(86) International application number: PCT/JP2010/006317
(87) International publication number: WO 2011/052186

(57) **Abstract**

A fluoranthene compound represented by the formula (1): 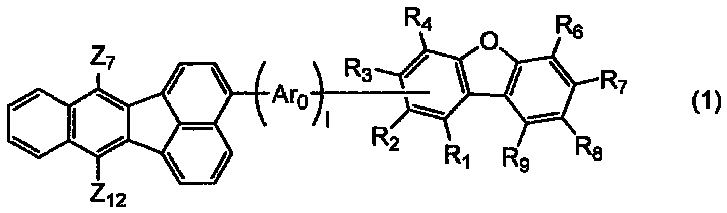 wherein Z₇ and Z₁₂ are each aryl or heteroaryl; Ar₀ is a single bond, aryl, or heteroaryl; R₁ to R₄ and R₆ to R₉ are each independently a hydrogen atom, alkyl, cycloalkyl, aryl, heteroaryl, or the like; and I is an integer of 1 to 4.

## Description

### TECHNICAL FIELD

The invention relates to a fluoranthene compound, an organic electroluminescence material-containing solution, and an organic electroluminescence device using the same. In particular, the invention relates to a fluoranthene compound capable of fabricating an organic electroluminescence device having a high luminous efficiency and a long life.

### BACKGROUND ART

An organic electroluminescence (EL) device is a self-emission device utilizing the principle that a fluorescent compound emits light by the recombination energy of holes injected from an anode and electrons injected from a cathode when an electric field is impressed. Such an organic EL device comprises a pair of electrodes, i.e. an anode and a cathode, and an organic light-emitting medium therebetween.

The organic light-emitting medium is formed of a stack of layers having each function. For example, it is a stack in which an anode, a hole-injecting layer, a hole-transporting layer, an emitting layer, and an electron-transporting layer and an electron-injecting layer are sequentially stacked.

As the emission material of the emitting layer, a material which emits light in each color (for example, red, green and blue) has been developed. For example, a fluoranthene compound is disclosed in Patent Document 1 and Patent Document 2 as a blue-emitting compound.
However, the fluoranthene compound disclosed in Patent Document 1 and Patent Document 2 has a problem that it is not satisfactory in respect of luminous efficiency and lifetime.

[Patent Document 1] JP-A-H10-189247
[Patent Document 2] JP-A-2005-068087

### DISCLOSURE OF THE INVENTION

An object of the invention is to provide a fluoranthene compound capable of fabricating an organic EL device having high luminous efficiency and long lifetime can be obtained.

According to the invention, the following fluoranthene compound, etc. are provided.
1. A fluoranthene compound represented by the formula (1): 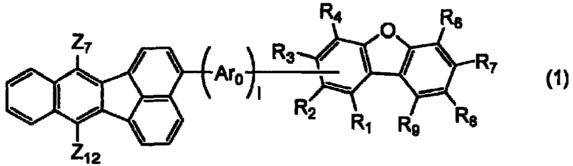 wherein
   Z₇ and Z₁₂ are independently a substituted or unsubstituted aryl group having 5 to 50 carbon atoms that form a ring (hereinafter referred to as the "ring carbon atoms"),, or a substituted or unsubstituted heteroaryl group having 5 to 50 atoms that form a ring (hereinafter referred to as the "ring atoms");
   Ar₀ is a single bond, a substituted or unsubstituted aryl group having 5 to 50 ring carbon atoms, or a substituted or unsubstituted heteroaryl group having 5 to 50 ring atoms, and Ar₀ is a linking group bonding to any one of R₁ to R₄ and R₆ to R₉;
   R₁ to R₄ and R₆ to R₉ are independently a hydrogen atom, an alkyl group, a cycloalkyl group, a substituted or unsubstituted aryl group, a substituted or unsubstituted heteroaryl group, or a substituted silyl group, or at least one pair of R₁ and R₂, R₂ and R₃, R₃ and R₄, R₆ and R₇, R₇ and R₈, and R₈ and R₉ independently bonds to each other to form a saturated or unsaturated ring structure which may have a further substituent; and
   I is an integer of 1 to 4; and
   when I is 2 or more, plural Ar₀s are the same or different, and substituents of adjacent Ar₀s may bond to each other.
2. The fluoranthene compound according to 1, wherein the fluoranthene compound is represented by the formula (2): 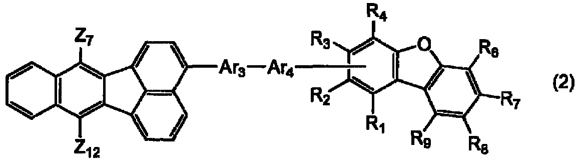 wherein
   Z₇, Z₁₂, R₁ to R₄, and R₆ to R₉ are the same as in the formula (1);
   Ar₃ and Ar₄ are independently a substituted or unsubstituted aryl group having 5 to 50 ring carbon atoms, or a substituted or unsubstituted heteroaryl group having 5 to 50 ring atoms, and Ar₄ is a linking group bonding to any one of R₁ to R₄ and R₆ to Rg; and
   a substituent of Ar₃ and a substituent of Ar₄ may bond(crosslink) to each other.
3. The fluoranthene compound according to 1, wherein the fluoranthene compound is represented by the formula (3): 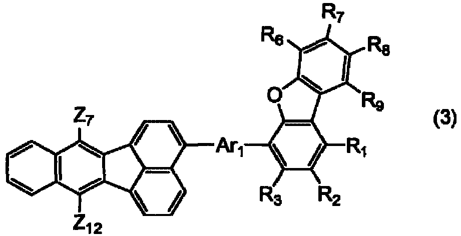 wherein
   Z₇, Z₁₂, R₁ to R₃, and R₆ to R₉ are the same as in the formula (1); and
   Ar₁ is a single bond, a substituted or unsubstituted aryl group having 5 to 50 ring carbon atoms, or a substituted or unsubstituted heteroaryl group having 5 to 50 ring atoms.
4. The fluoranthene compound according to 1, wherein the fluoranthene compound is represented by the formula (4): 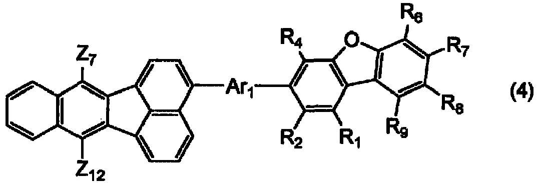 wherein
   Z₇, Z₁₂, R₁, R₂, R₄, and R₆ to R₉ are the same as in the formula (1); and
   Ar₁ is a single bond, a substituted or unsubstituted aryl group having 5 to 50 ring carbon atoms, or a substituted or unsubstituted heteroaryl group having 5 to 50 ring atoms.
5. The fluoranthene compound according to 1, wherein the fluoranthene compound is represented by the formula (5): 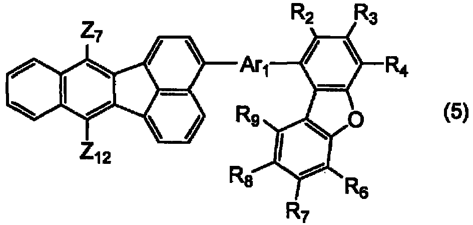 wherein
   Z₇, Z₁₂, R₂ to R₄, and R₆ to R₉ are the same as in the formula (1); and
   Ar₁ is a single bond, a substituted or unsubstituted aryl group having 5 to 50 ring carbon atoms, or a substituted or unsubstituted heteroaryl group having 5 to 50 ring atoms.
6. The fluoranthene compound according to 1, wherein the fluoranthene compound is represented by the formula (6): 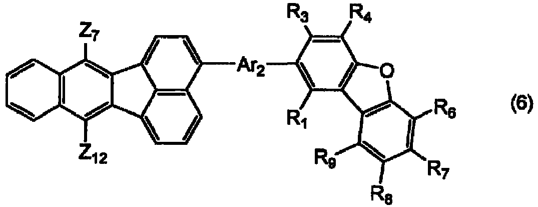 wherein
   Z₇, Z₁₂, R₁, R₃, R₄, and R₆ to R₉ are the same as in the formula (1); and
   Ar₂ is a single bond, a substituted or unsubstituted aryl group having 5 to 50 ring carbon atoms, or a substituted or unsubstituted heteroaryl group haying 5 to 50 ring atoms.
7. The fluoranthene compound according to 6, wherein
   Ar₂ is a single bond, a substituted or unsubstituted heteroaryl group having 5 to 50 ring atoms, or one of linking groups represented by the following formulas; and
   when Ar₂ is a single bond, at least one of R₁, R₃, R₄, R₆, R₇, R₈ and R₉ is an alkyl group, a cycloalkyl group, a substituted or unsubstituted aryl group, a substituted or unsubstituted heteroaryl group, or a substituted silyl group. 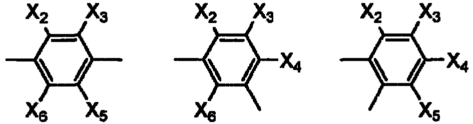 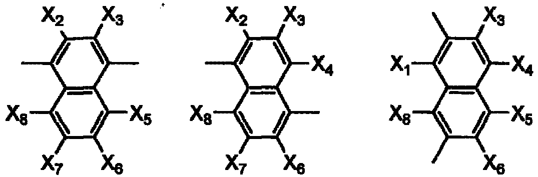 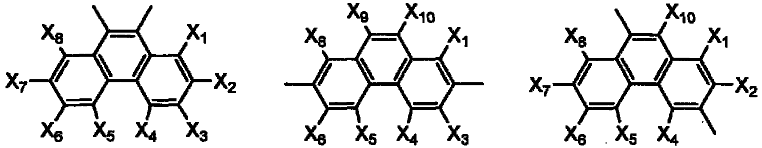 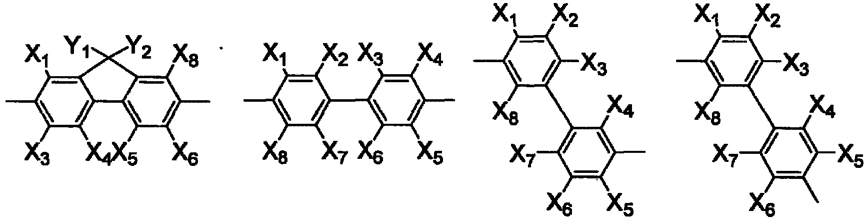 wherein
   X₁ to X₁₀, Y₁ and Y₂ are independently a hydrogen atom, a fluorine atom, a cyano group, an alkyl group, a cycloalkyl group, a substituted or unsubstituted aryl group, a substituted or unsubstituted heteroaryl group, or a substituted silyl group.
8. The fluoranthene compound according to any one of 1 to 7, wherein
   Z₇ and Z₁₂ are independently a phenyl group, a naphthyl group, a fluorenyl group, a 9,9'-dimethylfluorenyl group, a diethylfluorenyl group, a dipropylfluorenyl group, a diisopropylfluorenyl group, a dibutylfluorenyl group, a diphenylfluorenyl group, or a phenanthryl group.
9. An organic electroluminescence device which comprises:
   a pair of electrodes, and
   one or a plurality of organic compound layers comprising at least an emitting layer between the pair of electrodes, wherein
   at least one of the organic compound layers comprises at least one of the fluoranthene compound according to any one of 1 to 8.
10. The organic electroluminescence device according to 9, wherein the emitting layer comprises the fluoranthene compound.
11. The organic electroluminescence device according to 10, wherein the content of the fluoranthene compound in the emitting layer is 0.01 to 20 mass %.
12. The organic electroluminescence device according to 10 or 11, wherein the emitting layer further comprises a compound having an anthracene central skeleton represented by the formula (2a): 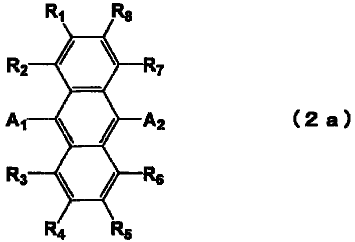 wherein
   A₁ and A₂ are independently a group derived from a substituted or unsubstituted aromatic ring having 6 to 20 ring carbon atoms, and the aromatic ring may be substituted by one or two or more substituents;
   the substituent is a substituted or unsubstituted aryl group having 6 to 50 ring carbon atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group, or a hydroxy group;
   when the aromatic ring is substituted by two or more substituents, the substituents may be the same or different, and adjacent substituents may bond to each other to form a saturated or unsaturated ring structure; and
   R₁ to R₈ are independently a hydrogen atom, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group, or a hydroxy group.
13. The organic electroluminescence device according to 12, wherein A₁ and A₂ are substituents different from each other.
14. The organic electroluminescence device according to 12, wherein at least one of A₁ and A₂ is a substituent having a substituted or unsubstituted fused ring group having 10 to 30 ring atoms.
15. The organic electroluminescence device according to 14, wherein the substituted or unsubstituted fused ring group having 10 to 30 ring atoms is a substituted or unsubstituted naphthalene ring.
16. The organic electroluminescence device according to 10 or 11, wherein the emitting layer further comprises a compound having a pyrene central skeleton represented by the formula (2b): 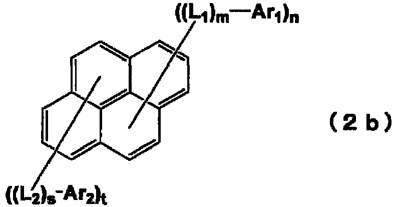 wherein
   Ar₁ and Ar₂ are independently a substituted or unsubstituted aryl group having 6 to 50 ring carbon atoms;
   L₁ and L₂ are independently a substituted or unsubstituted phenylene group, a substituted or unsubstituted naphthalene group, a substituted or unsubstituted fluorenylene group, or a substituted or unsubstituted dibenzosilolylene group;
   m is an integer of 0 to 2, n is an integer of 1 to 4, s is an integer of 0 to 2, and t is an integer of 0 to 4; and
   L₁ or Ar₁ bonds to one of the 1- to 5-positions of the pyrene, and L₂ or Ar₂ bonds to one of the 6- to 10-positions of the pyrene.
17. The organic electroluminescence device according to 10 or 11, wherein the emitting layer further comprises a compound having a triphenylamine skeleton represented by the formula (2c): 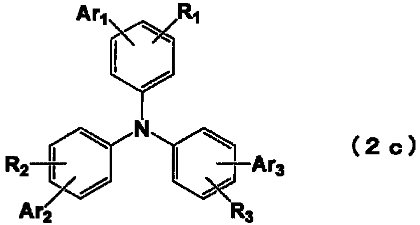 wherein
   Ar₁, Ar₂ and Ar₃ are independently a group having an anthrathene structure, a group having a phenanthrene structure, or a group having a pyrene structure; and
   R₁, R₂ and R₃ are independently a hydrogen atom or a substituent
18. The organic electroluminescence device according to 10 or 11, wherein the emitting layer further comprises a compound represented by the formula (2d): 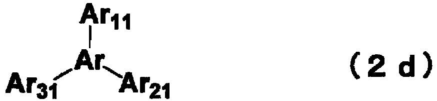 wherein
   Ar₁₁, Ar₂₁ and Ar₃₁ are independently an aryl group having 6 to 50 ring carbon atoms;
   the aryl group may be substituted by one or two or more substituents;
   at least one of Ar₁₁, Ar₂₁ and Ar₃₁, and the substituents of these aryl groups has a fused aryl structure having 10 to 20 ring carbon atoms or a fused heteroaryl structure having 6 to 20 ring carbon atoms; and
   Ar is a trivalent group derived from an aromatic ring or a heteroaromatic ring.
19. An organic electroluminescence material-containing solution which comprises:
   the fluoranthene compound according to any one of 1 to 8 which is an organic electroluminescence material, and
   a solvent
20. The organic electroluminescence material-containing solution according to 19, wherein
   the organic electroluminescence material comprises a host material and a dopant material;
   the dopant material is the fluoranthene compound according to any one of 1 to 8; and
   the host material is at least one selected from the compounds represented by the formula (2a) according to 12, the compound represented by the formula (2b) according to 16, the compound represented by the formula (2c) according to 17, and the compound represented by the formula (2d) according to 18.

According to the invention, a fluoranthene compound capable of fabricating an organic EL device which has a high luminous efficiency and a long life can be provided.
Also, according to the invention, an organic EL device having a high luminous efficiency and a long life can be provided.

### MODE FOR CARRYING OUT THE INVENTION

The fluoranthene compound of the invention is represented by the formula (1): 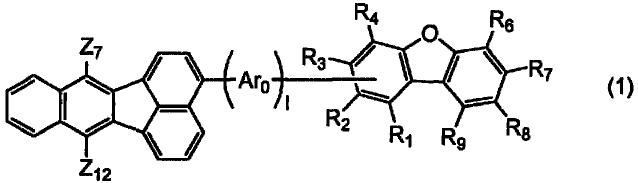 In the formula, Z₇ and Z₁₂ are independently a substituted or unsubstituted aryl group having 5 to 50 ring carbon atoms, or a substituted or unsubstituted heteroaryl group having 5 to 50 ring atoms.
Ar₀ is a single bond, a substituted or unsubstituted aryl group having 5 to 50 ring carbon atoms, or a substituted or unsubstituted heteroaryl group having 5 to 50 ring atoms, and Ar₀ is a linking group bonding to any one of R₁ to R₄ and R₆ to R₉. Here, when any one of R₁ to R₄ and R₆ to R₉ which bonds to Ar₀ is a hydrogen atom, the bond between Ar₀ and the dibenzofuran skeleton is a single bond.
R₁ to R₄ and R₆ to R₉ are independently a hydrogen atom, an alkyl group, a cycloalkyl group, a substituted or unsubstituted aryl group, a substituted or unsubstituted heteroaryl group, or a substituted silyl group, or at least one pair of R₁ and R₂, R₂ and R₃, R₃ and R₄, R₆ and R₇, R₇ and R₈, and R₈ and R₉ independently bond to each other to form a saturated or unsaturated ring structure which may have a further substituent; and
I is an integer of 1 to 4; and
when I is 2 or more, plural Ar₀s are the same or different, and substituents of adjacent Ar₀s may bond to each other.

In the benzofluoranthene compounds known so far, the conjugation length in the benzofluoranthene skeleton which mainly contributes to emission is long, and the planarity of the skeleton is high, so that stacking is likely to occur and may result in decrease in luminous efficiency.
On the contrary, in the fluoranthene compound of the invention, it is considered that introduction of the dibenzofuran skeleton having an oxygen atom around the benzofluoranthene skeleton brings about an effect to prevent the fluoranthene compound from assembly due to stacking, whereby the luminous efficiency is improved.

As mentioned above, R₁ and R₂, R₂ and R₃, R₃ and R₄, R₆ and R₇, R₇ and R₈, and R₈ and R₉ may independently bond to each other to form a saturated or unsaturated ring structure.
Examples of the ring structures include: 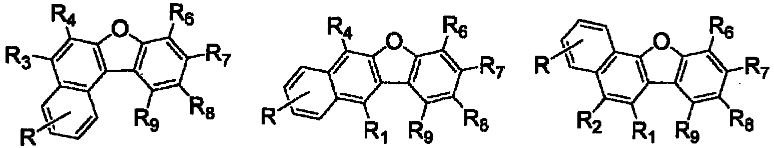 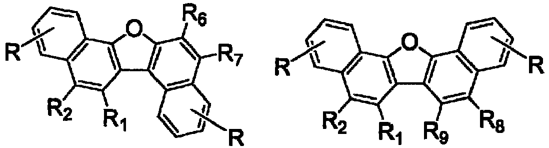 In the formulas, R is a hydrogen atom, a fluorine atom, a substituted silyl group, a cyano group, a substituted or unsubstituted aryl group, a substituted or unsubstituted heteroaryl group, an alkyl group or a cycloalkyl group.

When the number of Ar₀ is 2 or more, adjacent Ar₀s may be the same or different, the adjacent Ar₀s may crosslink to each other via a substituent of one of the Ar₀s, and substituent of the Ar₀s may crosslink to each other.
For example, when I is 2 and both of the two Ar₀s are phenylene groups, the crosslinked structure includes: 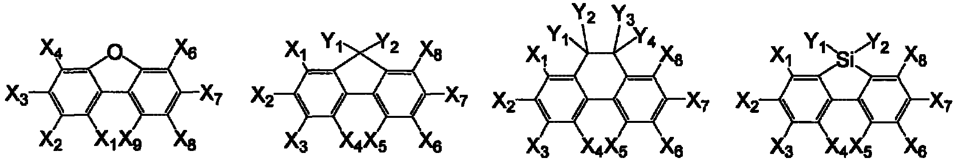 In the formulas, X₁ to X₉ and Y₁ to Y₄ are independently a hydrogen atom, a fluorine atom, a substituted silyl group, a cyano group, a substituted or unsubstituted aryl group, a substituted or unsubstituted heteroaryl group, an alkyl group or a cycloalkyl group. The bonds to the benzofluoranthene skeleton, and any one of R₁ to R₄ and R₆ to R₉ are formed at the positions of X₁ to X₉ and Y₁ to Y₄.
Here, when each of X₁ to X₉ and Y₁ to Y₄ which is bonded to the benzofluoranthene skeleton or any one of R₁ to R₄ and R₆ to R₉ is a hydrogen atom, the bond to the benzofluoranthene skeleton or any one of R₁ to R₄ and R₆ to R₉ is a single bond. When any one of R₁ to R₄ and R₆ to R₉ which bonds to X₁ to X₉ and Y₁ to Y₄ is a hydrogen atom, the bond between Ar₀ and the dibenzofuran skeleton is a single bond.

The fluoranthene compound represented by the formula (1) is preferably fluoranthene compounds represented by the following formulas (2), (3), (4), (5), and (6).

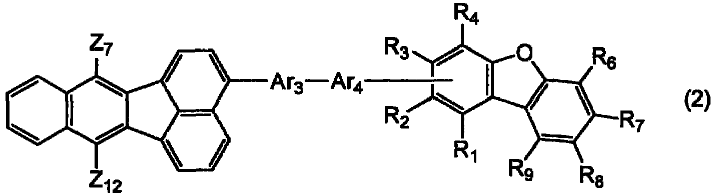

In the formula, Z₇, Z₁₂, R₁ to R₄, and R₆ to R₉ are the same as in the formula (1).
Ar₃ and Ar₄ are independently a substituted or unsubstituted aryl group having 5 to 50 ring carbon atoms, or a substituted or unsubstituted heteroaryl group having 5 to 50 ring atoms, and Ar₄ is a linking group bonding to any one of R₁ to R₄ and R₆ to R₉.
Ar₃ and Ar₄ may crosslink to each other via a substituent of either Ar₃ or Ar₄, and a substituent of Ar₃ and a substituent of Ar₄ may crosslink to each other.
Here, when any one of R₁ to R₄ and R₆ to R₉ which is bonded to the crosslinking structure formed by Ar₃ and Ar₄ is a hydrogen atom, the bond between the crosslinking structure and the dibenzofuran structure is a single bond.

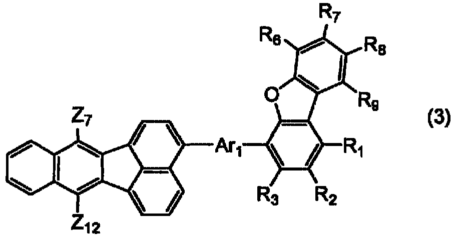

In the formula, Z₇, Z₁₂, R₁ to R₃, and R₆ to R₉ are the same as in the formula (1).
Ar₁ is a single bond, a substituted or unsubstituted aryl group having 5 to 50 ring carbon atoms, or a substituted or unsubstituted heteroaryl group having 5 to 50 ring atoms.

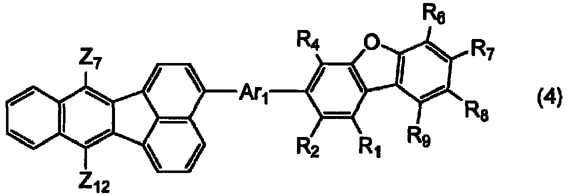

In the formula, Z₇, Z₁₂, R₁, R₂, R₄, and R₆ to R₉ are the same as in the formula (1).
Ar₁ is a single bond, a substituted or unsubstituted aryl group having 5 to 50 ring carbon atoms, or a substituted or unsubstituted heteroaryl group having 5 to 50 ring atoms.

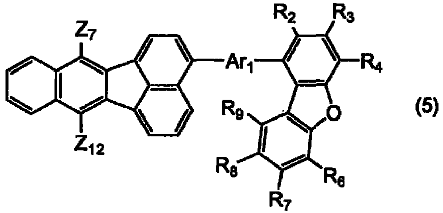

In the formula, Z₇, Z₁₂, R₂ to R₄, and R₆ to R₉ are the same as in the formula (1).
Ar₁ is a single bond, a substituted or unsubstituted aryl group having 5 to 50 ring carbon atoms, or a substituted or unsubstituted heteroaryl group having 5 to 50 ring atoms.

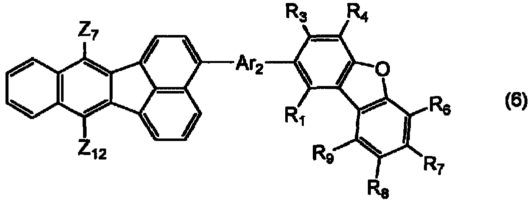

In the formula, Z₇, Z₁₂, R₁, R₃, R₄, and R₆ to R₉ are the same as in the formula (1).
Ar₂ is a single bond, a substituted or unsubstituted aryl group having 5 to 50 ring carbon atoms, or a substituted or unsubstituted heteroaryl group having 5 to 50 ring atoms.

In the fluoranthene compound represented by the formula (6), Ar₂ is preferably a single bond, a substituted or unsubstituted heteroaryl group having 5 to 50 ring atoms, or any one of linking groups represented by the following formulas. When Ar₂ is a single bond, at least one of R₁, R₃, R₄, R₆, R₇, R₈ and R₉ is an alkyl group, a cycloalkyl group, a substituted or unsubstituted aryl group, a substituted or unsubstituted heteroaryl group, or a substituted silyl group. 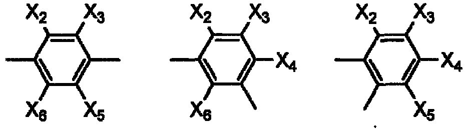 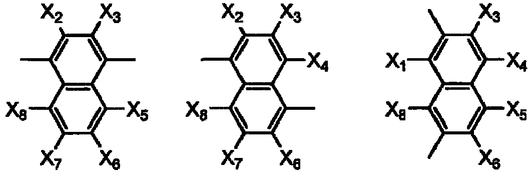 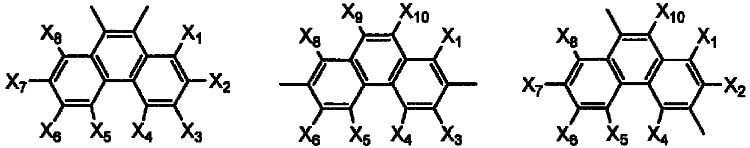 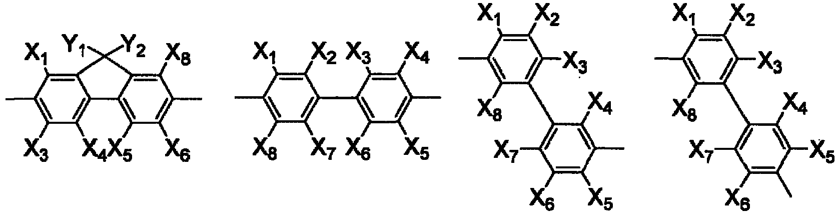 In the formulas, X₁ to X₁₀, Y₁ and Y₂ are independently a hydrogen atom, a fluorine atom, a substituted silyl group, a cyano group, a substituted or unsubstituted aryl group, a substituted or unsubstituted heteroaryl group, an alkyl group, or a cycloalkyl group. Here, a bonding line having no symbol indicates a single bond to the fluoranthene skeleton or the dibenzofuran skeleton.

In the fluoranthene compound represented by the formula (6), when Ar₂ is a single bond, at least one of R₁, R₃, R₄, R₆, R₇, R₈ and R₉ is a substituted aryl group, a substituted heteroaryl group, or a substituted silyl group, a substituent which further substitutes these substituents is preferably a substituted silyl group, an alkyl group, a cycloalkyl group, an aryl group, or a heteroaryl group.

Now, each of the substituents of the fluoranthene compound of the invention will be explained below.
Here, in the invention, the term "an aryl group" indicates "a group derived from an aromatic compound from which at least one hydrogen atom is removed", and includes not only an aryl group of monovalent but also "an arylene group" of divalent and the like. The same meaning is applied to "a heteroaryl group".
In the invention, "a hydrogen atom" includes deuterium and tritium.

The substituted or unsubstituted aryl group having 5 to 50 ring carbon atoms in Z₇, Z₁₂ and Ar₀ to Ar₄ is preferably a substituted or unsubstituted aryl group having 5 to 20 ring carbon atoms, and more preferably a substituted or unsubstituted aryl group having 5 to 14 ring carbon atoms. Examples thereof include a phenyl group, a naphthyl group, a phenanthryl group, an anthryl group, a benzanthryl group, a pyrenyl group, a chrysenyl group, a fluorenyl group, a 9,9-dimethylfluorenyl group, a diethylfluorenyl group, a dipropylfluorenyl group, a diisopropylfluorenyl group, a dibutylfluorenyl group, a diphenylfluorenyl group, a biphenyl group, and a triphenylene group. Preferred are a phenyl group, a naphthyl group, a phenanthryl group, a biphenyl group and a 9,9-dimethylfluorenyl group.

The substituted or unsubstituted heteroaryl group having 5 to 50 ring atoms in Z₇, Z₁₂ and Ar₀ is preferably a substituted or unsubstituted heteroaryl group having 5 to 20 ring atoms, and more preferably a substituted or unsubstituted heteroaryl group having 5 to 14 ring atoms. Examples thereof include a pyrrolyl group, a pyrazinyl group, a pyridinyl group, an indolyl group, an isoindolyl group, a furyl group, a dibenzofuranyl group, a benzofuranyl group, an isobenzofuranyl group, a quinolyl group, an isoquinolyl group, a quinoxalinyl group, a carbozolyl group, a phenanthridinyl group, an acridinyl group, a phenanthrolinyl group, a phenazinyl group, a phenothiazinyl group, a phenoxazinyl group, an oxazolyl group, a benzoxazolyl group, an oxaziazolyl group, a furazanyl group, thienyl group, a thiophenyl group, a benzothiophenyl group, a dibenzothiophenyl group, an imidazolyl group, a benzoimidazolyl group, a pyralozinyl group and piperidinyl group. Preferred are a pyrazinyl group, a pyridinyl group, a quinolyl group, an isoquinolyl group, a dibenzofuranyl group, and a benzoxazolyl group.

The substituted or unsubstituted aryl group in R₁ to R₄, R₆ to R₉, X₁ to X₁₀, and Y₁ to Y₄ includes the same substituents in the above-mentioned substituted or unsubstituted aryl group having 5 to 50 ring carbon atoms.
The substituted or unsubstituted heteroaryl group in R₁ to R₄, R₆ to R₉, X₁ to X₁₀ and Y₁ to Y₄ includes the same substituents as the above-mentioned substituted or unsubstituted heteroaryl group having 5 to 50 ring atoms.

The alkyl group in R₁ to R₄, R₆ to Rg, X₁ to X₁₀ and Y₁ to Y₄ includes a methyl group, an ethyl group, a propyl group, an isopropyl group, a n-butyl group, a s-butyl group, an isobutyl group, a t-butyl group, a n-pentyl group, a n-hexyl group, a n-heptyl group, and a n-octyl group. Preferred are a methyl group, an ethyl group, a propyl group, an isopropyl group, a n-butyl group, a s-butyl group, an isobutyl group, and a t-butyl group, and particularly preferred are a methyl group, an ethyl group, a propyl group, an isopropyl group, and a t-butyl group.

The cycloalkyl group in R₁ to R₄, R₆ to R₉, X₁ to X₁₀ and Y₁ to Y₄ includes a cyclopropyl group, a cyclobutyl group, a cyclopentyl group, a cyclohexyl group, a 4-methylcyclohexyl group, a 1-adamantyl group, a 2-adamantyl group, a 1-norbomyl group, and a 2-norbornyl group. Preferred are a cyclopentyl group and a cyclohexyl group.

The substituted silyl group in R₁ to R₄, R₆ to R₉, X₁ to X₁₀ and Y₁ to Y₄ includes a trimethylsilyl group, a triethylsilyl group, a t-butyldimethylsilyl group, a vinyldimethylsilyl group, a propyldimethylsilyl group, a triphenylsilyl group, and a triisopropylsilyl group. Preferred are a trimethylsilyl group, a triethylsilyl group, and a t-butyldimethylsilyl group.

The substituent which further substitutes the substituent of the above-mentioned fluoranthene compound (for example, as the substituent, the substituents in the substituted or unsubstituted aryl group having 5 to 50 ring carbon atoms may be mentioned, and the substituent is applied to all the substituents in the expression of "substituted or unsubstituted") includes a fluorine atom, a substituted silyl group, a cyano group, a substituted or unsubstituted aryl group, a substituted or unsubstituted heteroaryl group, an alkyl group, and a cycloalkyl group. Preferred are a substituted silyl group, a substituted or unsubstituted aryl group, an alkyl group, and a cycloalkyl group. (Substitution repeats.)
Examples of these substituents are as mentioned above.

Examples of the fluoranthene compound of the invention will be shown below.

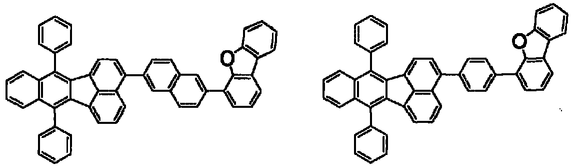

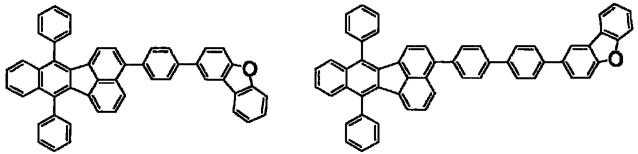

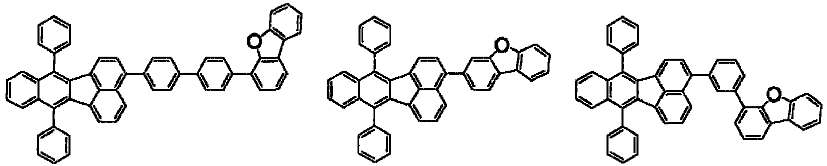

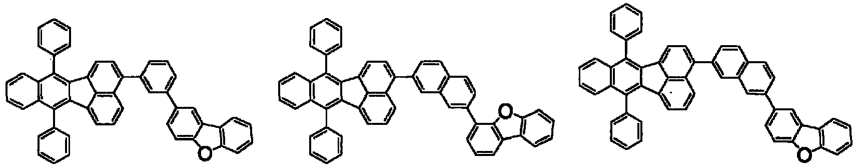

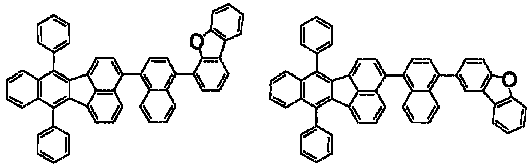

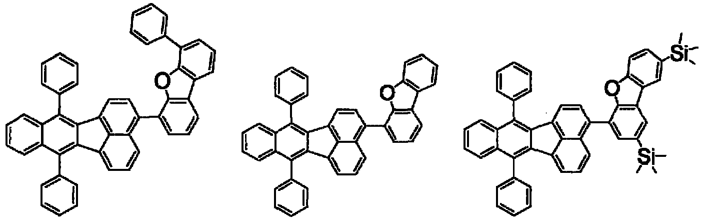

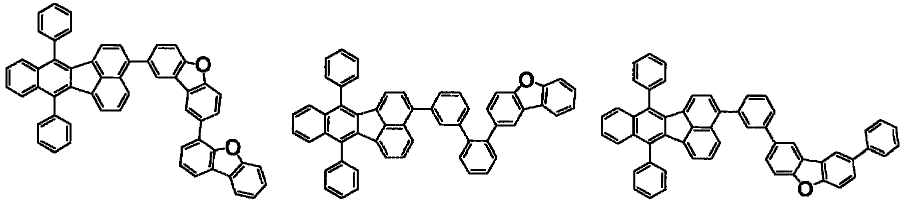

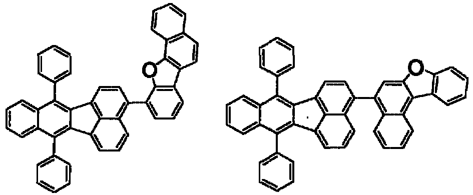

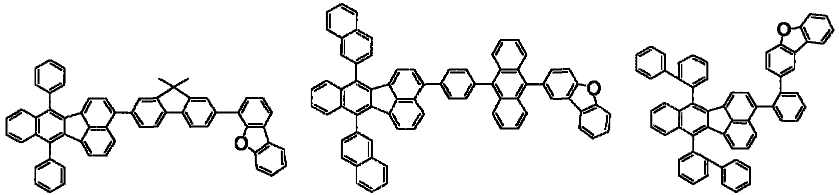

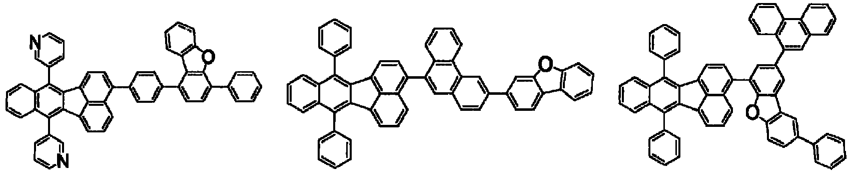

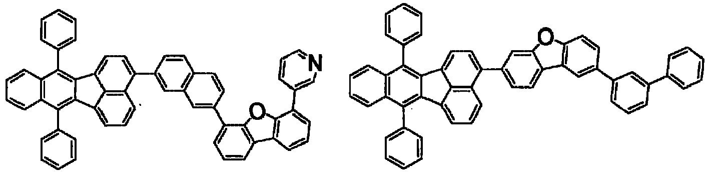

The fluoranthene compound of the invention can be synthesized by a method described in J. Org. Chem., 55,4190 (1990), J. Org. Chem., 68, 883 (2003) or by a carbon-carbon bond generation reaction (Suzuki reaction, Kumada-Tamao coupling reaction, Still reaction, Sonogashira reaction, or the like) and an annulation reaction.

It is preferred that the fluoranthene compound of the invention be used as a material for an organic EL device. It is particularly preferable to use it as an emitting material for an organic EL device, especially as a doping material.

Regarding the organic EL device of the invention, in an organic electroluminescence device in which organic compound layers comprising one layer or a plurality of layers containing at least an emitting layer between a pair of electrodes, at least one of the above-mentioned organic compound layers comprises the fluoranthene compound of the invention.
In the organic EL device of the invention, it is preferred that the emitting layer contain a fluoranthene compound and that the emitting layer contain the fluoranthene compound of the invention preferably in an amount of 0.1 to 20 wt%, further preferably 0.5 to 20 wt%, particularly preferably 1 to 18 wt% and most preferably 2.5 to 15 wt%.
the organic EL device using the material for an organic EL device containing the fluoranthene compound of the invention can emit blue light.

When the fluoranthene compound of the invention is used as an emitting material of the organic EL device, it is preferred that the emitting layer contain at least one kind of the fluoranthene compound and at least one kind selected from the compounds represented by the general formulas (2a), (2b), (2c) and (2d). It is preferred that at least one kind selected from the compounds represented by the following general formulas (2a), (2b), (2c) and (2d) be a host material.

An explanation will be made on the compounds represented by the formulas (2a), (2b), (2c) and (2d).

### Formula (2a)

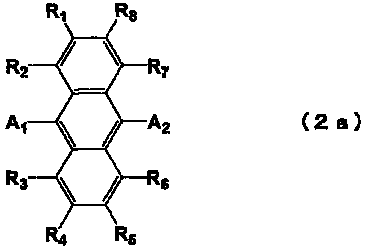

In the formula (2a), A₁ and A₂ are independently a group induced from a substituted or unsubstituted aromatic ring having 6 to 20 ring carbon atoms. The aromatic ring may be substituted by one or two or more substituents. The substituent is selected from a substituted or unsubstituted aryl group having 6 to 50 ring carbon atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group and a hydroxyl group. If the above-mentioned aromatic ring is substituted by two or more substituents, the substituents may be the same or different, and adjacent substituents may be bonded together to form a saturated or unsaturated ring structure.
R₁ to R₈ are independently selected from a hydrogen atom, a substituted or unsubstituted aryl group having 6 to 50 ring carbon atoms, a substituted or unsubstituted heterocyclic group having 5 to 50 ring atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group and a hydroxyl group.

In the formula (2a), it is preferred that A₁ and A₂ mentioned above be different groups.
In the formula (2a), it is preferred that at least one of A₁ and A₂ be a substituent having a substituted or unsubstituted fused ring group having 10 to 30 ring atoms.
It is preferred that the above-mentioned substituted or unsubstituted fused ring group having 10 to 30 ring atoms be a naphthalene ring.

The substituted or unsubstituted aryloxy group and arylthio group having 5 to 50 ring atoms for R₁ to R₈ and the substituent of the aromatic ring in the formula (2a) are represented by ―OY' and ―SY", respectively. Examples of ―Y' and Y" include the same examples as those for the substituted or unsubstituted aryl group having 6 to 50 ring atoms of the substituent of R₁ to R₈ and the aromatic ring.
The substituted or unsubstituted alkoxycarbonyl group having 1 to 50 carbon atoms for R₁ to R₈ and the substituent of the aromatic ring in the formula (2a) is represented by -COOZ. Examples of Z include the same examples as those of the substituted or unsubstituted alkyl group having 1 to 50 carbon atoms for R₁ to R₈ and the substituent of the aromatic ring.

Examples of the silyl group for R₁ to R₈ and the substituent of the aromatic ring in the formula (2a) include a trimethylsilyl group, a triethylsilyl group, a t-butyldimethylsilyl group, a vinyldimethylsilyl group, a propyldimethylsilyl group and a triphenylsilyl group.
As the halogen atom for R₁ to R₈ and the substituent of the aromatic ring in the formula (2a), fluorine or the like can be given.
As the substituent for R₁ to R₈ and the substituent for the aromatic ring, a halogen atom, a hydroxyl group, a nitro group, a cyano group, an alkyl group, an aryl group, a cycloalkyl group, an alkoxy group, an aromatic heterocyclic group, an aralkyl group, an aryloxy group, an arylthio group, an alkoxycarbonyl group, a carboxy group or the like can be given.

It is preferred that the anthracene derivative represented by the formula (2a) be a compound having a structure shown by the following formula (2a'). 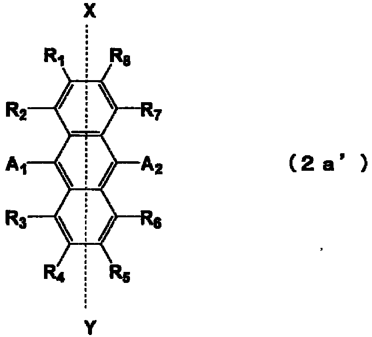 In the formula (2a'), A₁ and A₂, R₁ to R₈ are independently the same as that in the formula (2a), and the same specific examples can be given,
provided that groups do not symmetrically bond to 9- and 10-positions of the central anthracene with respect to X-Y axis.

Specific examples of the anthracene derivative to be used in the organic EL device of the invention, represented by the formula (2a) include known various anthracene derivatives such as those having two anthracene skeletons in the molecule shown in JP-A-2004-356033, [0043] to [0063] and compounds having one anthracene skeleton shown in WO2005/061656, pages 27 to 28.

### Formula (2b)

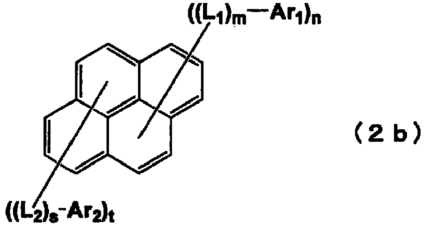

In the formula (2b), Ar₁ and Ar₂ are independently a substituted or unsubstituted aryl group having 6 to 50 ring carbon atoms.
L₁ and L₂ are independently a group selected from a substituted or unsubstituted phenylene group, a substituted or unsubstituted naphthalenylene group, a substituted or unsubstituted fluorenylene group and a substituted or unsubstituted dibenzosilolylene group.
m is an integer of 0 to 2, n is an integer of 1 to 4, s is an integer of 0 to 2 and t is an integer of 0 to 4.
L₁ or Ar₁ bonds to any position of the 1^{st} to 5^{th} positions of pyrene, and L₂ or Ar₂ bonds to any position of the 6^{th} to 10^{th} positions of pyrene.

L₁ and L₂ in the formula (2b) are preferably selected from a substituted or unsubstituted phenylene group and a substituted or unsubstituted fluorenylene group.
As the substituent thereof, substituents similar to those exemplified in the above-mentioned aromatic ring group can be given.
m in the formula (2b) is preferably an integer of 0 to 1, and n in the formula (2b) is preferably an integer of 1 to 2. s in the formula (2b) is preferably an integer of 0 to 1.
t in the formula (2b) is preferably an integer of 0 to 2.

### Formula (2c)

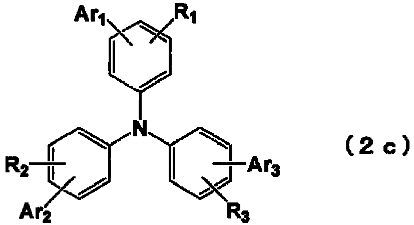

In the formula (2c), Ar₁, Ar₂ and Ar₃ are independently selected from a group having an anthracene structure, a group having a phenanthrene structure, a group having a perylene structure and a group having a pyrene structure.
R₁, R₂ and R₃ are independently a hydrogen atom or a substituent.

Ar₁, Ar₂ and Ar₃ in the formula (2c) is preferably selected from a substituted or unsubstituted anthrylphenyl group, an anthryl group, a phenanthrenyl group, a perylenyl group and a pyrenyl group, more preferably selected from an alkyl-substituted or unsubstituted anthrylphenyl group and a pyrenyl group, and particularly preferably selected from a pyrenyl group and a phenanthrenyl group.

Examples of R₁, R₂ and R₃ in the formula (2c) include a hydrogen atom, an alkyl group (preferably one having 1 to 30 carbon atoms, more preferably 1 to 20 carbon atoms, and particularly preferably 1 to 10 carbon atoms; specific examples thereof include methyl, ethyl, isopropyl, t-butyl, n-octyl, n-decyl, n-hexadecyl, cyclopropyl, cyclopentyl and cyclohexyl), an alkenyl group (preferably one having 2 to 30 carbon atoms, more preferably 2 to 20 carbon atoms, and particularly preferably 2 to 10 carbon atoms; specific examples thereof include vinyl, allyl, 2-butenyl and 3-pentenyl), an alkynyl group (preferably one having 2 to 30 carbon atoms, more preferably 2 to 20 carbon atoms, and particularly preferably 2 to 10 carbon atoms; specific examples thereof include propargyl and 3-pentynyl), an aryl group (preferably one having 6 to 30 carbon atoms, more preferably 6 to 20 carbon atoms, and particularly preferably one having 6 to 12 carbon atoms; the specific examples thereof include phenyl, p-methylphenyl, naphthyl and anthranyl), an amino group (preferably one having 0 to 30 carbon atoms, more preferably 0 to 20 carbon atoms, and particularly preferably 0 to 10 carbon atoms; the specific examples thereof include amino, methylamino, dimethylamino and diethylamino, dibenzylamino, diphenylamino and ditolylamino group), an alkoxy group (preferably one having 1 to 30 carbon atoms, more preferably 1 to 20 carbon atoms, and particularly preferably 1 to 10 carbon atoms; the specific examples thereof include methoxy, ethoxy, butoxy, and 2-ethylhexyloxy), an aryloxy group (preferably one having 6 to 30 carbon atoms, more preferably 6 to 20 carbon atoms, and particularly preferably 6 to 12 carbon atoms; the specific examples thereof include phenyloxy, 1-naphthyloxy and 2-naphthyloxy), a heteroaryloxy group (preferably one having 1 to 30 carbon atoms, more preferably 1 to 20 carbon atoms, and particularly preferably 1 to 12 carbon atoms; the specific examples thereof include pyridyloxy, pyrazyloxy, pyrimidyloxy and quinolyloxy); an acyl group (preferably one having 1 to 30 carbon atoms, more preferably 1 to 20 carbon atoms, and particularly preferably 1 to 12 carbon atoms; the specific examples thereof include acetyl, benzoyl, formyl and pivaloyl), an alkoxycarbonyl group (preferably one having 2 to 30 carbon atoms, more preferably 2 to 20 carbon atoms, and particularly preferably 2 to 12 carbon atoms; the specific examples thereof include methoxycarbonyl and ethoxycarbonyl); an aryloxycarbonyl group (preferably one having 7 to 30 carbon atoms, more preferably 7 to 20 carbon atoms and particularly preferably 7 to 12 carbon atoms; the specific examples thereof include phenyloxycarbonyl); an acyloxy group (preferably one having 2 to 30 carbon atoms, more preferably 2 to 20 carbon atoms and particularly preferably 2 to 10 carbon atoms; the specific examples thereof include acetoxy and benzoyloxy), an acylamino group (preferably one having 2 to 30 carbon atoms, more preferably 2 to 20 carbon atoms, and particularly preferably 2 to 10 carbon atoms; the specific examples thereof include acetylamino and benzoylamino), an alkoxycarbonylamino group (preferably one having 2 to 30 carbon atoms, more preferably 2 to 20 carbon atoms, and particularly preferably 2 to 12 carbon atoms; the specific examples thereof include methoxycarbonylamino), an aryloxycarbonylamino group (preferably one having 7 to 30 carbon atoms, more preferably 7 to 20 carbon atoms, particularly preferably 7 to 12 carbon atoms; the specific examples thereof include phenyloxycarbonylamino), a sulfonylamino group (preferably one having 1 to 30 carbon atoms, more preferably one having 1 to 20 carbon atoms and particularly preferably one having 1 to 12 carbon atoms; the specific examples thereof include methanesulfonylamino and benzenesulfonylamino), a sulfamoylamino group (preferably one having 0 to 30 carbon atoms, more preferably 0 to 20 carbon atoms and particularly preferably 0 to 12 carbon atoms; the specific examples thereof include sulfamoyl, methylsulfamoyl, dimethylsulfamoyl and phenylsulfamoyl), a carbamoyl group (preferably one having 1 to 30 carbon atoms, more preferably one having 1 to 20 carbon atoms and particularly preferably one having 1 to 12 carbon atoms; the specific examples thereof include carbamoyl, methylcarbamoyl, diethylcarbamoyl and phenylcarbamoyl), an alkylthio group (preferably one having 1 to 30 carbon atoms, more preferably 1 to 20 carbon atoms and particularly preferably 1 to 12 carbon atoms; the specific examples include methylthio and ethylthio), an arylthio group (preferably one having 6 to 30 carbon atoms, more preferably one having 6 to 20 carbon atoms and particularly preferably one having 6 to 12 carbon atoms; the specific examples thereof include phenylthio), a heteroarylthio group (preferably one having 1 to 30 carbon atoms, more preferably one having 1 to 20 carbon atoms, and particularly preferably one having 1 to 12 carbon atoms; the specific examples thereof include pyridylthio, 2-benzoimidazolylthio, 2-benzoxazolylthio and 2-benzothiazolylthio); a sulfonyl group (preferably one having 1 to 30 carbon atoms, more preferably 1 to 20 carbon atoms and particularly preferably 1 to 12 carbon atoms; the specific examples thereof include mesyl and tosyl); a sulfinyl group (preferably one having 1 to 30 carbon atoms, more preferably 1 to 20 carbon atoms and particularly preferably 1 to 12 carbon atoms; the specific examples thereof include methanesulfinyl and benzenesulfinyl), an ureido group (preferably one having 1 to 30 carbon atoms, more preferably 1 to 20 carbon atoms and particularly preferably 1 to 12 carbon atoms; the specific examples thereof include ureido, methylureido and phenylureido), a phosphoric amide group (preferably one having 1 to 30 carbon atoms, more preferably 1 to 20 carbon atoms and particularly preferably 1 to 12 carbon atoms; the specific examples thereof include diethylphosphoric amide and phenylphosphatoric amide), a hydroxyl group, a mercapto group, a halogen atom (for example, a fluorine atom, a chlorine atom, a bromine atom, an iodine atom or the like can be given), a cyano group, a sulfo group, a carboxy group, a nitro group, a hydroxamic acid group, a sulfino group, a hydrazino group, an imino group, a heterocyclic group (preferably one having 1 to 30 carbon atoms, more preferably 1 to 12 carbon atoms, and as the hetero atom, a nitrogen atom, an oxygen atom and a sulfur atom can be given, the specific examples thereof include imidazolyl, pyridyl, quinolyl, furyl, thienyl, piperidyl, morpholino, benzoxazolyl, benzimidazolyl and benzothiazolyl), and a substituted or unsubstituted silyl group (preferably one having 3 to 40 carbon atoms, more preferably 3 to 30 carbon atoms and particularly preferably 3 to 24 carbon atoms; the specific examples thereof include trimethylsilyl and triphenylsilyl). These substituents may further be substituted.
R₁, R₂ and R₃ in the formula (2c) are preferably selected from an alkyl group and an aryl group.

Specific examples of the amine derivative to be used in the organic EL device of the invention represented by the formula (2c) include known various amine derivatives such as those shown in JP-A-2002-324678 [0079] to [0083].

### Formula (2d)

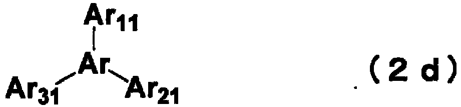

In the formula (2d), Ar₁₁, Ar₂₁ and Ar₃₁ are independently an aryl group having 6 to 50 ring carbon atoms. The aryl group may be substituted by one or two or more substituents.
At least one of Ar₁₁, Ar₂₁ and Ar₃₁ and the substituents of these aryl groups has a fused ring aryl structure having 10 to 20 ring carbon atoms or a fused ring heteroaryl structure having 6 to 20 ring carbon atoms.
Ar is a trivalent group induced from the aromatic ring or the heterocyclic aromatic ring.

The aryl group having 6 to 50 ring carbon atoms of Ar₁₁, Ar₂₁ and Ar₃₁ in the formula (2d) preferably has 6 to 30, more preferably 6 to 20, further preferably 6 to 16 ring carbon atoms. These aryl groups may further have a substituent

Examples of the substituent on the aryl group include an alkyl group, an alkenyl group, an alkynyl group, an aryl group, an amino group, an alkoxy group, an aryloxy group, a heteroaryloxy group, an acyl group, an alkoxycarbonyl group, an aryloxycarbonyl group, an acyloxy group, an acylamino group, an alkoxycarbonylamino group, an aryloxy carbonylamino group, a sulfonylamino group, a sulfamoyl group, a carbamoyl group, an alkylthio group, an arylthio group, a heteroarylthio group; a sulfonyl group, a sulfinyl group, an ureido group, a phosphoric amide group, a hydroxy group, a mercapto group, a halogen atom (for example, a fluorine atom, a chlorine atom, a bromine atom, an iodine atom, etc. can be given), a cyano group, a sulfo group, a carboxy group, a nitro group, a hydroxamic acid group, a sulfino group, a hydrazino group, an imino group, a heterocyclic group, a silyl group, etc. can be given. These substituents may be further substituted.

As the fused ring aryl structure having 10 to 20 ring carbon atoms of at least one of Ar₁₁, Ar₂₁, Ar₃₁ and the substituent of these aryl groups in the formula (2d), a naphthalene structure, an anthracene structure, a phenanthrene structure, a pyrene structure and a perylene structure or the like can be given. Of these, a naphthalene structure, an anthracene structure, a pyrene structure and a phenanthrene structure are preferable. A phenanthrene structure and an aryl structure with four or more rings are preferable, with a pyrene structure being particularly preferable.
As the fused ring heteroaryl structure having 6 to 20 ring carbon atoms of Ar₁₁, Ar₂₁, Ar₃₁ and the substituent of these aryl groups in the formula (2d), a quinoline structure, a quinoxaline structure, a quinazoline structure, an acrylidine structure, a phenanthridine structure, a phthalazine structure, a phenanthroline structure or the like can be given. Of these, a quinoline structure, a quinoxaline structure, a quinazoline structure, a phthalazine structure and a phenanthroline structure are preferable.

A trivalent group induced from the aromatic ring of Ar in the formula (2d) preferably has 6 to 30 carbon atoms, more preferably 6 to 20 carbon atoms and further preferably 6 to 16 carbon atoms.
The trivalent group induced from the heterocyclic aromatic ring of Ar in the formula (2d) preferably contains an atom selected from a nitrogen atom, a sulfur atom and an oxygen atom as the hetero atom. More preferably it contains a nitrogen atom.

In the organic EL device of the invention, each organic layer such as the emitting layer or the like can be formed by a dry film forming method such as the vacuum vapor deposition method, the molecular beam epitaxy (MBE) method, sputtering, plasma and ion plating and a coating method such as spin coating, dipping, casting, bar coating, roll coating, flow coating, ink jetting or the like of a solution.
In particular, when an organic EL device is fabricated by using the fluoranthene compound of the invention, the organic compound layer and the emitting layer can be formed not only by deposition but also by a wet method.
Although there are no particular restrictions on the film thickness of each layer of the organic compound layer, it is required to set it to a suitable film thickness. Generally, if the film thickness is too small, pinholes or the like are generated, and a sufficient luminance may not be obtained even though an electric field is applied. On the other hand, if the film thickness is too large, a high voltage is required to be applied in order to obtain a certain optical output, resulting in a poor efficiency. In general, a suitable film thickness is in the range of 5 nm to 10 µm, with the range of 10 nm to 0.2 µm being further preferable.

In the case of the wet film forming method, as the material for an organic EL device, an organic EL material containing solution which contains the fluoranthene compound of the invention and a solvent can be used. It is preferable to use an organic EL material containing solution containing the fluoranthene compound of the invention and at least one selected from the compounds shown by the formulas (2a), (2b), (2c) and (2d).
In this case, an organic EL material forming each layer is dissolved or dispersed in a suitable solvent to prepare a solution containing an organic EL material to form a thin film. Any solvent may be used. Examples of the solvent include halogen-based hydrocarbon-based solvents such as dichloromethane, dichloroethane, chloroform, carbon tetrachloride, tetrachloroethane, trichloroethane, chlorobenzene, dichlorobenzene, chlorotoluene and trifluorotoluene; an ether-based solvent such as dibutyl ether, tetrahydrofuran, tetrahydropyrane, dioxane, anisole and dimethoxyethane, an alcohol-based solvent such as methanol, ethanol, propanol, isopropanol, butanol, pentanol, hexanol, cyclohexanol, methylcellosolve, ethylcellosolve and ethylene glycol, a ketone-based solvent such as acetone, methyl ethyl ketone, diethylketone, 2-hexanone, methylisobutylketone, 2-heptanone, 4-heptanone, diisobutylketone, acetonylacetone, isophorone, cyclohexanone, methylhexanone and acetophenone, a hydrocarbon-based solvent such as benzene, toluene, xylene, ethylbenzene, hexane, cyclohexane, octane, decane and tetralin, an ester-based solvent such as ethyl acetate, butyl acetate and amyl acetate, a branched carbonate ester-based solvent such as dimethyl carbonate, methyl ethyl carbonate and diethyl carbonate, and a cyclic carbonate ester-based solvent such as ethylene carbonate and propylene carbonate. Of these, a hydrocarbon-based solvent or an ether-based solvent such as toluene and dioxane are preferable. Further, these solvents may be used singly or in combination of two or more. Usable solvents are not limited thereto.

In each organic compound layer, a suitable resin or additive may be used for improvement of film-forming properties, prevention of pinhole generation in the film or the like. Usable resins include insulating resins such as polystyrene, polycarbonate, polyarylate, polyester, polyamide, polyurethane, polysulfone, polymethyl methacrylate, polymethyl acrylate and cellulose, and copolymers thereof, photoconductive resins such as poly-N-vinylcarbazole and polysilane, and conductive resins such as polyaniline, polythiophene and polypyrrole. As the additive, antioxidants, UV absorbers, plasticizers or the like can be given.
In order to improve stability to temperature, humidity, atmosphere or the like of the organic EL device of the invention, it is possible to provide a protective layer on the surface of the device, or to protect the entire device with silicone oil, a resin or the like.
In the organic EL device of the invention, it is preferred that a layer selected from a calcogenide layer, a metal halide layer and a metal oxide layer on at least one surface of the pair of electrode.

### (Constitution of Organic EL device)

### (1) Structure of organic EL device

The representative device structure of the organic EL device of the invention is given below.
(1) Anode/emitting layer/cathode.
(2) Anode/hole-injecting layer/emitting later/cathode
(3) Anode/emitting layer/electron-injecting layer/cathode
(4) Anode/hole-injecting layer/emitting layer/electron-injecting layer/cathode
(5) Anode/organic semiconductor layer/emitting layer/cathode
(6) Anode/organic semiconductor layer/electron blocking layer/emitting layer/cathode
(7) Anode/organic semiconductor layer/emitting layer/adhesion-improving layer/cathode
(8) Anode/hole-injecting layer/hole-transporting layer/emitting layer/electron-injecting layer/cathode
(9) Anode/insulating layer/emitting layer/insulating layer/cathode
(10) Anode/inorganic semiconductor layer/insulating layer/emitting layer/insulating layer/cathode
(11) Anode/organic semiconductor layer/insulating layer/emitting layer/insulating layer/cathode
(12) Anode/insulating layer/hole-injecting layerlhole-transporting layer/emitting layer/insulating layer/cathode
(13) Anode/insulating layer/hole-injecting layer/hole-transporting layer/emitting layer/electron-injecting layer/cathode
Of these, the structure (8) is preferably used.
The fluoranthene compound of the invention may be used in any of the above-mentioned organic layers. However, it is preferred that it be contained in the emission region or in the hole-transporting region of these constituent elements.

### (2) Transparent substrate

The organic EL device is formed on a transparent substrate. The transparent substrate as referred to herein is a substrate for supporting the organic EL device, and is preferably a flat and smooth substrate having a 400-to-700-nm-visible-light transmittance of 50% or more.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfone, and polysulfone.

### (3) Anode

The anode of the organic EL device plays a role for injecting holes into its hole-transporting layer or emitting layer. The anode effectively has a work function of 4.5 eV or more. Indium tin oxide alloy (ITO), tin oxide (NESA), gold, silver, platinum, copper, and the like may be used as the material for the anode. As the anode, in order to inject electrons into the electron-transporting layer or the emitting layer, a material having a small work function is preferable.
The anode can be formed by forming these electrode materials into a thin film by vapor deposition, sputtering or the like.
In the case where emission from the emitting layer is outcoupled through the anode, the transmittance of the anode to the emission is preferably more than 10%. The sheet resistance of the anode is preferably several hundred Ω/ or less. The film thickness of the anode, which varies depending upon the material thereof, is usually selected from 10 nm to 1 µm, preferably from 10 to 200 nm.

### (4) Emitting layer

The emitting layer of the organic EL device has the following functions (1), (2) and (3) in combination. That is,
(1) Injection function: function of allowing injection of holes from the anode or hole-injecting layer and injection of electrons from the cathode or electron-injecting layer upon application of an electric field
(2) Transporting function: function of moving injected carriers (electrons and holes) due to the force of an electric field
(3) Emitting function: function of allowing electrons and holes to recombine to emit light Note that electrons and holes may be injected into the emitting layer with different degrees, or the transportation capabilities indicated by the mobility of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film.
The molecular deposition film as referred to herein means a thin film which is formed by deposition of a raw material compound in the vapor-phase state or a film which is formed by solidification of a raw material compound in the solution state or in the liquid-phase state and is distinguished from a thin film (molecular accumulation film) formed using the LB method by the difference in aggregation structure or higher order structure or the difference in function due to the difference in structure.
The emitting layer may also be formed by dissolving a binder such as a resin and a material compound in a solvent to obtain a solution, and forming a thin film from the solution by spin coating or the like.
When using the fluoranthene compound of the invention for the emitting layer, the fluoranthene compound of the invention can be used as both of the dopant material and the host material. However, it is particularly preferably used as the dopant material.
In the invention, if desired, known emitting materials other than the emitting materials formed of the compound of the invention having a fluoranthene structure and a fused ring containing compound may be contained in the emitting layer insofar as the object of the invention is not impaired. An emitting layer containing other known emitting materials may be stacked on the emitting layer containing the emitting materials of the invention.
The thickness of an emitting layer is preferably from 5 to 50 nm, more preferably from 7 to 50 nm and most preferably from 10 to 50 nm. When it is less than 5 nm, the formation of an emitting layer and the adjustment of chromaticity may become difficult. When it exceeds 50 nm, the driving voltage may increase.

### (5) Hole-injecting/transporting Layer (Hole-transporting zone)

The hole-injecting/transporting layer is a layer for helping the injection of holes into the emitting layer to transport the holes to a light emitting region. The hole mobility thereof is large and the ionization energy thereof is usually as small as 5.5 eV or less. Such a hole-injecting/transporting layer is preferably made of a material which can transport holes to the emitting layer at a low electric field intensity. The hole mobility thereof is preferably at least 10⁻⁴ cm²/V·second when an electric field of, e.g. 10⁴ to 10⁶ V/cm is applied.
If the fluoranthene compound is used in the hole-transporting region, the hole-injecting/transporting layer may be formed by using the fluoranthene compound alone or in a mixture with other materials.
As the material for forming the hole-injecting/transporting layer in a mixture with the fluoranthene compound of the invention, any materials which have the above preferable properties can be used as the material for forming the hole-injecting/transporting layer without particular limitation. The material for forming the hole-injecting/transporting layer can be arbitrarily selected from materials which have been widely used as a material transporting carriers of holes in photoconductive materials and known materials used in a hole-injecting transporting layer of organic EL devices.

Specific examples thereof include a triazole derivative, an oxadiazole derivative, and an imidazole derivative, a polyarylalkane derivative, a pyrazoline derivative, and a pyrazolone derivative, a phenylene diamine derivative, an arylamine derivative, an amino-substituted chalcone derivative, an oxazole derivative, a styrylanthracene derivative, a fluorenone derivative, a hydrazone derivative, a stilbene derivative, a silazane derivative, a polysilane-based copolymer and an aniline-based copolymer.

Although the above-mentioned materials are used as the material for the hole-injecting/transporting layer, a porphyrin compound, an aromatic tertiary amine compound and a styrylamine compound are preferable, with an aromatic tertiary amine compound being preferable.
It is preferable to use a compound having two fused aromatic rings in the molecule thereof, for example, 4,4'-bis(N-(1-naphthyl)-N-phenylamino)biphenyl (abbreviated by NPD, hereinafter), and 4,4',4"-tris(N-(3-methylphenyl)-N-phenylamino)triphenylamine (abbreviated by MTDATA, hereinafter) wherein three triphenylamine units are linked in a star-burst form.
In addition to the aromatic dimethylidene type compounds mentioned above as the material for an emitting layer, inorganic compounds, p-type Si and p-type SiC can also be used as the material of the hole-injecting layer.
The hole-injecting/transporting layer can be formed from the above-mentioned compounds by a known method such as vacuum vapor deposition, spin coating, casting or LB technique. The film thickness of the hole-injecting/transporting layer is not particularly limited, and is usually from 5 nm to 5 µm.

### (6) Electron-injecting layer

The electron-injecting layer is a layer which assists injection of electrons into the emitting layer, and exhibits a high electron mobility. An adhesion-improving layer is a type of the electron-injecting layer formed of a material which exhibits excellent adhesion to the cathode. The material used in the electron-injecting layer is preferably a metal complex of 8-hydroxyquinoline or a derivative thereof.
As specific examples of a metal complex of an 8-hydroxyquinoline or a derivative thereof, metal chelate oxynoid compounds including a chelate of oxine (generally, 8-quinolinol or 8-hydroxyquinoline) can be given.
For example, Alq described as the emitting material can be used for the electron-injecting layer.

An electron-transmitting compound of the following formula can be given as the oxadiazole derivative.

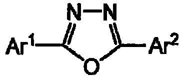

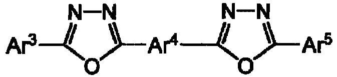

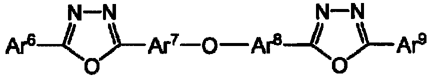

wherein Ar¹, Ar², Ar³, Ar⁵, Ar⁶, and Ar⁹ are independently substituted or unsubstituted aryl groups and may be the same or different. Ar⁴, Ar⁷, and Ar⁸ are independently substituted or unsubstituted arylene groups and may be the same or different.
The electron-transmitting compound is preferably one from which a thin film can be formed.

A preferred embodiment of the invention is a device containing a reducing dopant in an electron-transferring region or in an interfacial region between the cathode and the organic layer. The reducing dopant is defined as a substance which can reduce an electron-transferring compound. Accordingly, various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.
More specific examples of the preferred reducing dopants include at least one alkali metal selected from the group consisting of Li (work function: 2.9 eV), Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). One having a work function of 2.9 eV or less is particularly preferable. Among these, a more preferable reducing dopant is at least one alkali metal selected from the group consisting of K, Rb and Cs. Even more preferable is Rb or Cs. Most preferable is Cs. These alkali metals are particularly high in reducing ability. Thus, the addition of a relatively small amount thereof to an electron-injecting zone improves the luminance of the organic EL device and makes the lifetime thereof long. As a reducing agent having a work function of 2.9 eV or less, combinations of two or more alkali metals are preferable, particularly combinations including Cs, such as Cs and Na, Cs and K, Cs and Rb, or Cs, Na and K are preferable. The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By forming the electron-injecting layer, current leakage can be effectively prevented and electron-injecting properties can be improved. As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved. Specifically preferable alkali metal calcogenides include Li₂O, K₂O, Na₂S, Na₂Se and Na₂O and preferable alkaline earth metal calcogenides include CaO, BaO, SrO, BeO, BaS and CaSe. Preferable halides of alkali metals include LiF, NaF, KF, LiCl, KCl and NaCl. Preferable halides of alkaline earth metals include fluorides such as CaF₂, BaF₂, SrF₂, MgF₂ and BeF₂ and the halides other than the fluorides.

Semiconductors forming an electron-transporting layer include one or combinations of two or more of oxides, nitrides, and oxidized nitrides containing at least one element of Ba, Ca, Sr, Yb, Al, Ga, In, Li, Na, Cd, Mg, Si, Ta, Sb and Zn. An inorganic compound forming an electron-transporting layer is preferably a microcrystalline or amorphous insulating thin film. When the electron-transporting layer is formed of the insulating thin films, more uniformed thin film is formed whereby pixel defects such as a dark spot are decreased. Examples of such an inorganic compound include the above-mentioned alkali metal calcogenides, alkaline earth metal calcogenides, halides of alkali metals, and halides of alkaline earth metals.

### (7) Cathode

As the cathode, a metal having a small work function (4 eV or less), an alloy, an electroconductive compound or a mixture thereof are used as an electrode material in order to inject electrons to an electron-injecting/transporting layer Specific examples of the electrode substance include sodium, sodium-potassium alloy, magnesium, lithium, magnesium/silver alloy, aluminum/aluminum oxide, aluminum/lithium alloy, indium, and rare earth metals.
This cathode can be formed by making the electrode substances into a thin film by vapor deposition, sputtering or some other method.
In the case where light is emitted from the emitting layer through the cathode, the cathode preferably has a light transmittance of larger than 10%.
The sheet resistance of the cathode is preferably several hundreds Ω/ or less, and the film thickness thereof is usually from 10 nm to 1 µm, preferably from 50 to 200 nm.

### (8) Insulating layer

In the organic EL device, pixel defects based on leakage or a short circuit are easily generated since an electric field is applied to the super thin film. In order to prevent this, it is preferred to insert an insulating thin layer between the pair of electrodes.
Examples of the material used in the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide, and vanadium oxide.
A mixture or laminate thereof may be used.

### (9) Method for forming an organic EL device

The organic EL device can be fabricated by forming an anode, an emitting layer, optionally a hole-injecting layer, and optionally an electron-injecting layer, and further forming a cathode using the materials and methods exemplified above. The organic EL device can be fabricated in the order reverse to the above, i.e., the order from a cathode to an anode.
An example of the fabrication of the organic EL device will be described below wherein the following layers are successively formed on a transparent substrate: anode/hole-injecting layer/emitting layer/electron-injecting layer/cathode.
First, a thin film made of an anode material is formed into a thickness of 1 µm or less, preferably 10 to 200 nm on an appropriate transparent substrate by vacuum vapor deposition, sputtering or some other method, thereby forming an anode. Next, a hole-injecting layer is formed on this anode. As described above, the hole-injecting layer can be formed by vacuum vapor deposition, spin coating, casting, LB technique, or some other method. Vacuum vapor deposition is preferred since a homogenous film is easily obtained and pinholes are not easily generated. In the case where the hole-injecting layer is formed by vacuum vapor deposition, conditions for the deposition vary depending upon a compound used (a material for the hole-injecting layer), a desired crystal structure or recombining structure of the hole-injecting layer, and others. In general, the conditions are preferably selected from the following: deposition source temperature of 50 to 450°C, vacuum degree of 10⁻⁷ to 10⁻³ torr, deposition rate of 0.01 to 50 nm/second, substrate temperature of -50 to 300°C, and film thickness of 5 nm to 5 µm.

The emitting layer can also be formed on the hole-injecting layer by making a desired organic luminescent material into a thin film by vacuum vapor deposition, sputtering, spin coating, casting or some other method. Vacuum vapor deposition is preferred since a homogenous film is easily obtained and pinholes are not easily generated. In the case where the emitting layer is formed by vacuum vapor deposition, conditions for the deposition, which vary depending on a compound used, can be generally selected from conditions similar to those for the hole-injecting layer.
Next, an electron-injecting layer is formed on this emitting layer. Like the hole-injecting layer and the emitting layer, the layer is preferably formed by vacuum vapor deposition because a homogenous film is required to be obtained. Conditions for the deposition can be selected from conditions similar to those for the hole-injecting layer and the emitting layer.
The compound of the invention, depending on the layer where it is contained, i.e. the emission region or the hole-transporting region, can be co-deposited with other materials when vacuum vapor deposition is used. If the spin coating method is used, it can be contained by mixing with other materials.
Lastly, a cathode is stacked thereon to obtain an organic EL device.
The cathode is made of a metal, and deposition or sputtering may be used. However, vacuum vapor deposition is preferred in order to protect underlying organic layers from being damaged when the cathode film is formed.
For the organic EL device fabrication that has been described above, it is preferred that the formation from the anode to the cathode be continuously carried out, using only one vacuuming operation.

The film thickness of each of the organic layers in the organic EL device of the invention is not particularly limited. In general, defects such as pinholes are easily generated when the film thickness is too small. Conversely, when the film thickness is too large, a high applied voltage becomes necessary, leading to low efficiency. Usually, the film thickness is preferably in the range of several nanometers to one micrometer.
If a DC voltage is applied to the organic EL device, emission can be observed when the polarities of the anode and the cathode are positive and negative, respectively, and a DC voltage of 5 to 40 V is applied. When a voltage with an opposite polarity is applied, no electric current flows and hence, emission does not occur. If an AC voltage is applied, uniform emission can be observed only when the cathode and the anode have a positive polarity and a negative polarity, respectively. The waveform of the AC applied may be arbitrary.

### (Application of organic EL device)

The organic EL device of the invention can be applied to products which require a high luminous efficiency even at a low driving voltage. As application examples, a display apparatus, a display, a lighting apparatus, a printer light source, and the back light of a liquid crystal display, etc. can be given. It can also be applied to fields such as a sign, a signboard and interiors. As a display apparatus, an energy-saving, highly visible flat panel display can be given. Moreover, as a printer light source, the organic EL device can be used as a light source of a laser beam printer. Moreover, the volume of an apparatus can be reduced sharply by using the device of the invention. As for the lighting apparatus or the back light, energy-saving effects can be expectable by using the organic EL device of the invention.

### EXAMPLES

Now, the invention will be explained in detail based on examples. However, the invention is not limited to the following examples without departing from the scope and spirit of the invention.

### Synthesis Example 1

### [Synthesis of 5-bromoacenaphthylene]

29.2 g (128.7 mmol) of 2,3-dichloro-5,6-dicyano-1,4-benzoquinone (DDQ) was added to 25.4 g (107.3 mmol) of 5-bromoacenaphthene and 500 mL of anhydrous benzene, and the mixture was stirred with heat under reflux for 6 hours. To the reaction mixture, 6.0 g (26.4 mmol) of DDQ were further added, and stirred with heat for 4 hours. After the reaction mixture was left to cool, precipitates were removed by filtration and washed with chloroform. The filtrates were mixed together and washed with 10% aqueous sodium hydroxide solution and water. After liquid separation, the organic phase was dried with anhydrous sodium sulfate, and the solvent was distilled off. The resultant substance was dried under reduced pressure to obtain 13.0 g of 5-bromoacenaphthylene as a brown solid (yield: 51.6 %).

### Synthesis Example 2

### [Synthesis of 3-bromo-7,12-dibenzo[k]fluoranthene]

A mixture of 14.9 g (55.2 mmol) of 1,3-diphenylisobenzofuran, 12.8 g (55.2 mmol) of 5-bromoacenaphthylene synthesized in Synthesis Example 1 and 50 mL of toluene was stirred with. heat under reflux for 16 hours. After distillation of the solvent, 1200 mL of acetic acid was added, and the mixture was heated at a temperature of 80°C. To the mixture, 150 mL of 48% HBr aqueous solution was added, and the mixture was stirred at a temperature of 80°C for one hour. After cooling the mixture to room temperature, precipitates were obtained by filtration and washed with methanol. The resulting yellow solid was recrystallized from 200 mL of toluene. Crystals were obtained by filtration, and 19.8g of 3-bromo-7,12-dibenzo[k]fluoranthene as a yellow solid (yield: 74%).

### Synthesis Example 3

### [Synthesis of 7,12-dibenzo[k]fluoranthen-3-ylboronic acid]

30.8g (64.0 mmol) of 3-bromo-7,12-dibenzo[k]fluoranthene synthesized in Synthesis Example 2 was dissolved in 400 mL of anhydrous tetrahydrofuran and 300 mL of anhydrous toluene, and the solution was cooled to a temperature of -70°C. To the solution, 44.6 mL (70.4 mmol) of n-butyl lithium was dropwise added, and the mixture was stirred for one hour. To the mixture, 44.0 mL (192 mmol) of triisopropyl boronic acid ester was added, and allowed to warm to room temperature over two hours. Precipitates were obtained by filtration, washed with toluene, and dried under reduced pressure to obtain 25.14g of 7,12-diphenylbenzo[k]fluoranthen-3-ylboronic acid as a yellow solid (yield: 88%).

### Example 1

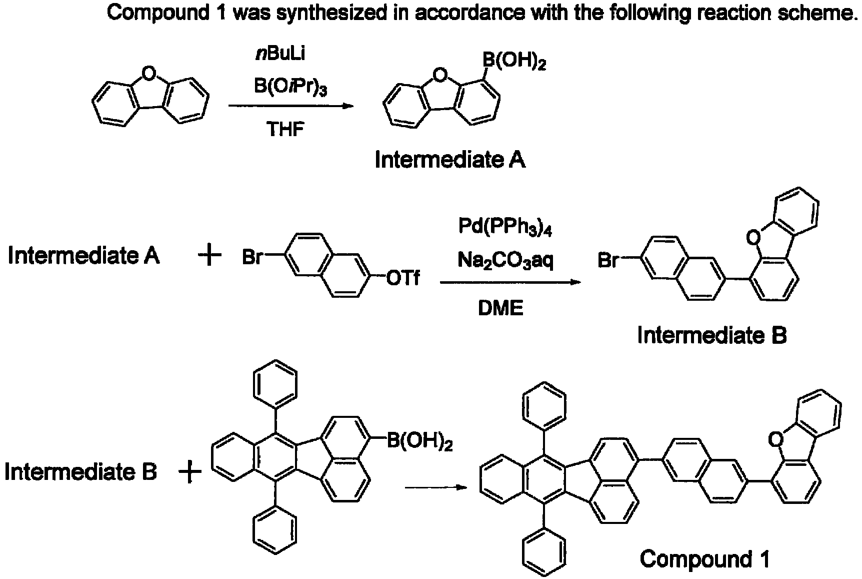

### [Synthesis of Intermediate A]

A 500 mL round bottom flask was charged with 20.5 g of dibenzofuran and 90 mL of anhydrous tetrahydrofuran under a flow of argon, and cooled to a temperature of -68°C. Then, 77.3 mL (1.57M) of a solution of n-butyl lithium in hexane was added to the flask, and the mixture was warmed to a temperature of -20°C, and stirred for one hour. The mixture was again cooled to a temperature of -68°C, and 83.4 mL of triisopropyl boronic acid ester was dropwise added thereto. Then, the temperature of the reaction mixture was gradually increased, and the reaction was carried out at room temperature for 5 hours. 3N hydrochloric acid and ethyl acetate were added to the reaction mixture, followed by liquid separation and extraction. Then, the organic phase was washed with clean water and saturated saline solution, dried with sodium sulfate and concentrated to obtain a crude product. The crude product was recrystallized from toluene. The resulting solid was dried under reduced pressure to obtain 16.1 g of a white solid. The white solid was identified as Intermediate A by the FD-MS (Field Desorption Mass Spectrometry) analysis.

### [Synthesis of Intermediate B]

4.0 g of Intermediate A, 7.1 g of 6-bromo-2-naphthyl trifluoromethanesulfonate, 660 mg of tetrakis(triphenylphosphine)palladium(O) [Pd(PPh₃)₄], 6.0 g (in 29 mL of clean water) of sodium carbonate and dimethoxyethane were added under a flow of argon, and the reaction was carried out under reflux for 7 hours. After cooling, the reaction solution was subjected to filtration. The resulting solid was washed with methanol and clean water. The solid was purified by silica gel chromatography (toluene/hexane (15/85)) and dried under reduced pressure to obtain 3.0 g of a white solid. The white solid was identified as Intermediate B by the FD-MS analysis.

### [Synthesis of Compound 1]

In the synthesis of Intermediate B, the reaction was carried out in the same manner except that 7,12-diphenylbenzo[k]fluoranthen-3-ylbaronic acid prepared in Synthesis Example 3 was used in place of Intermediate A, Intermediate B was used in place of 6-bromo-2-naphthyl trifluoromethanesulfonate, and a solvent mixture of toluene and dimethoxyethane was used in place of dimethoxyethane, to obtain

### Compound 1.

For the resulting Compound 1, the FDMS, and the maximum wavelength λmax of UV absorption and the maximum wavelength of fluorescence emission in a toluene solution of are indicated below.
FDMS, calcd for C54H32O=696, found m/z=696 (M+)
UV (PhMe); λmax, 423 nm, FL (PhMe, λex=380 nm); λmax, 441 nm

### Example 2

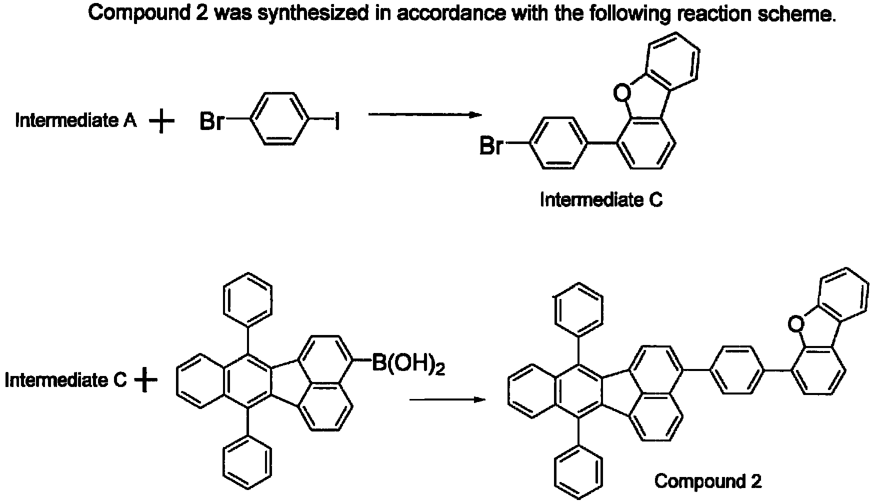

### [Synthesis of Intermediate C]

In the synthesis of Intermediate B in Example 1, the reaction was carried out in the same manner except that 1-bromo-4-iodobenzene was used in place of 6-bromo-2-naphthyl trifluoromethanesulfonate, to obtain Intermediate C.
The product was identified as Intermediate C by the FD-MS analysis.

### [Synthesis of Compound 2]

In the synthesis of Intermediate B in Example 1, the reaction was carried out in the same manner except that 7,12-diphenylbenzo[k]fluoranthen-3-ylboronic acid was used in place of Intermediate A, Intermediate C was used in place of 6-bromo-2-naphthyl trifluoromethanesulfonate, and a solvent mixture of toluene and dimethoxyethane was used in place of dimethoxyethane, to obtain Compound 2.
For the resulting compound 2, the FD-MS, and the maximum wavelength λmax of UV absorption and the maximum wavelength of fluorescence emission in a toluene solution are indicated below.
FDMS, calcd for C50H30O=646, found m/z=646 (M+)
UV (PhMe); Amax, 422 nm, FL (PhMe, λex=380 nm); λmax, 439 nm

### Example 3

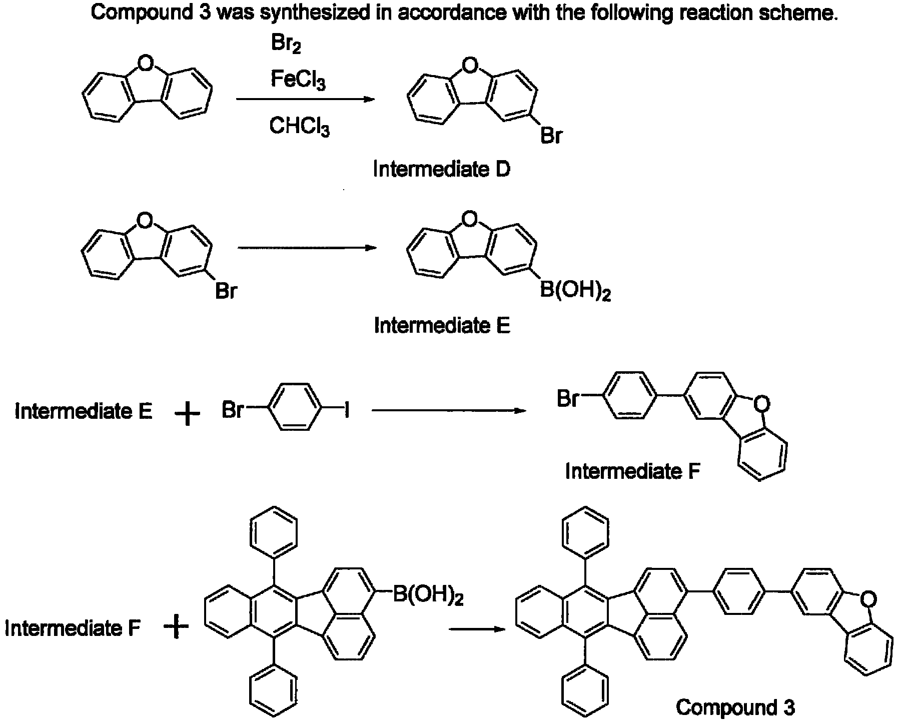

### [Synthesis of Intermediate D]

13.5 g of dibenzofuran, 389 mg of iron chloride (III) and 135 mL of anhydrous chloroform were added under a flow of argon, and the mixture was cooled to a temperature of 140°C. Then, 12.9 g of bromine was dropwise added to the mixture over 30 minutes. The mixture was gradually increased to room temperature and reacted for 5 hours. A saturated sodium sulfite aqueous solution was added to the reaction solution, followed by liquid separation. A crude product obtained by concentration of the organic layer was dissolved with super heat in hexane to precipitate crystals, followed by filtration. This recrystallization treatment was repeated four times, and activated carbon treatment was carried out. The resulting solid was dried under reduced pressure to obtain 8.2 g of a white solid. The white solid was identified as Intermediate D by the FD-MS analysis.

### [Synthesis of Intermediate E]

In the synthesis of Intermediate A in Example 1, the reaction was carried out in the same manner except that Intermediate D was used in place of dibenzofuran, to obtain Intermediate E.
The product was identified as Intermediate D by the FD-MS analysis.

### [Synthesis of Intermediate F]

In the synthesis of Intermediate B in Example 1, the reaction was carried out in the same manner except that Intermediate E was used in place of Intermediate A, and 1-bromo-4-iodobenzene was used in place of 6-bromo-2-naphthyl trifluoromethanesulfonate, to obtain Intermediate F.
The product was identified as Intermediate D by the FD-MS (Field Desorption Mass Spectrometry) analysis.

### [Synthesis of Compound 3]

In the synthesis of Intermediate B in Example 1, the reaction was carried out in the same manner except that 7,12-diphenylbenzo[k]fluoranthen-3-ylboronic acid was used in place of Intermediate A, Intermediate F was used in place of 6-bromo-2-naphthyl trifluoromethanesulfonate, and a solvent mixture of toluene and dimethoxyethane was used in place of dimethoxyethane, to obtain Compound 3.
For the resultant Compound 3, the FDMS, and the maximum wavelength λmax of UV absorption, and the maximum wavelength of fluorescence emission in a toluene solution are shown below.
FDMS, calcd for C50H300=646, found m/z=646 (M+)
UV (PhMe); λmax, 422 nm, FL (PhMe, λex=378 nm); λmax, 439 nm

### Example 4

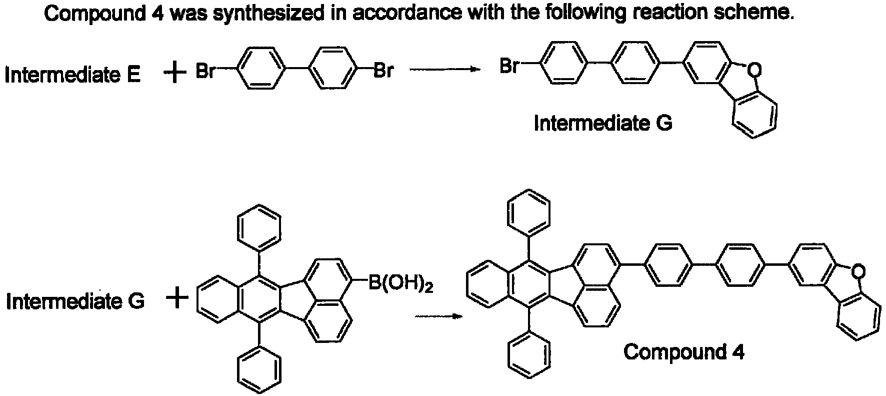

### [Synthesis of Intermediate G]

In the synthesis of Intermediate B in Example 1, the reaction was carried out in the same manner except that 4,4'-dibromobiphenyl was used in place of 6-bromo-2-naphthyl trifluoromethanesulfonate, and Intermediate E was used in place of Intermediate A, to obtain Intermediate G.
The product was identified as Intermediate G by the FD-MS analysis.

### [Synthesis of Compound 4]

In the synthesis of Intermediate B in Example 1, the reaction was carried out in the same manner except that 7,12-diphenylbenzo[k]fluoranthen-3-ylboronic acid was used in place of Intermediate A, Intermediate G was used in place of 6-bromo-2-naphthyl trifluoromethanesulfonate, and a solvent mixture of toluene and dimethoxyethane was used in place of dimethoxyethane, to obtain Compound 4.
For the resulting Compound 4, the FDMS, and the maximum wavelength λmax of UV absorption and the maximum wavelength of fluorescence emission in a toluene solution are shown.
FDMS, calcd for C56H34O=722, found m/z=722 (M+)
UV (PhMe); λmax, 423 nm, FL (PhMe, λex=380 nm); λmax, 440 nm

### Example 5

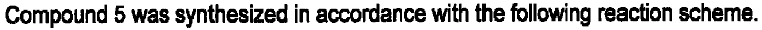

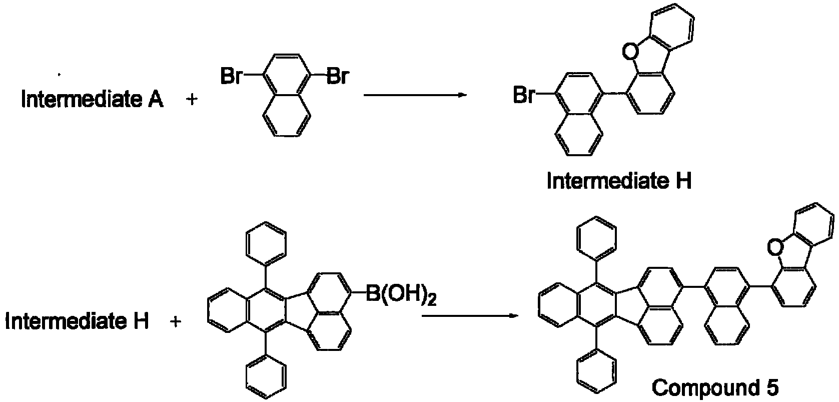

### [Synthesis of Intermediate H]

In the synthesis of Intermediate B in Example 1, the reaction was carried out in the same manner except that 1,4-dibromonaphthalene was used in place of 6-bromo-2-naphthyl trifluoromethanesulfonate, to obtain Intermediate H.
The product was identified as Intermediate H by the FD-MS analysis.

### [Synthesis of Compound 5]

In the synthesis of Intermediate B in Example 1, the reaction was carried out in the same manner except that 7,12-diphenylbenzo[k]fluoranthen-3-ylboronic acid was used in place of Intermediate A, Intermediate H was used in place of 6-bromo-2-naphthyl trifluoromethanesulfonate, and a solvent mixture of toluene and dimethoxyethane was used in place of dimethoxyethane, to obtain Compound 5.
For the resulting Compound 5, the FD-MS, and the maximum wavelength λ max of UV absorption and the maximum wavelength of fluorescence emission in a toluene solution are shown.
FDMS, calcd for C54H32O=696, found m/z=696 (M+)
UV (PhMe); λmax, 417 nm, FL (PhMe, λex=375 nm); λmax, 436 nm

### Example 6

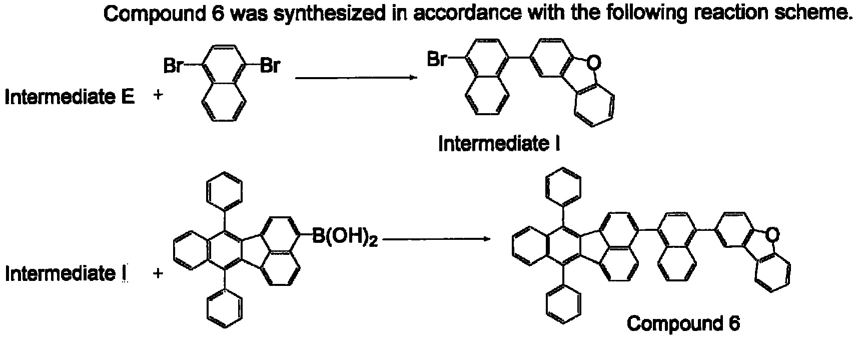

### [Synthesis of Intermediate I]

In the synthesis of Intermediate B in Example 1, the reaction was carried out in the same manner except that Intermediate E was used in place of Intermediate A, and 1,4-dibromonaphthalene was used in place of 6-bromo-2-naphthyl trifluoromethanesulfonate, to obtain Intermediate I.
The product was identified as Intermediate I by the FD-MS analysis.

### [Synthesis of Compound 6]

In the synthesis of Intermediate B in Example 1, the reaction was carried out in the same manner except that 7,12-diphenylbenzo[k]fluoranthen-3-ylboronic acid was used in place of Intermediate A, Intermediate I was used in place of 6-bromo-2-naphthyl trifluoromethanesulfonate, and a solvent mixture of toluene and dimethoxyethane was used in place of dimethoxyethane, to obtain Compound 6.
For the resulting Compound 6, the FDMS, and the maximum wavelength λmax of UV absorption and the maximum wavelength of fluorescence emission in a toluene solution are shown.
FDMS, calcd for C54H32O=696, found m/z=696 (M+)
UV (PhMe) ;λmax, 417 nm, FL (PhMe, λex=375 nm); λmax, 436 nm

### Example 7

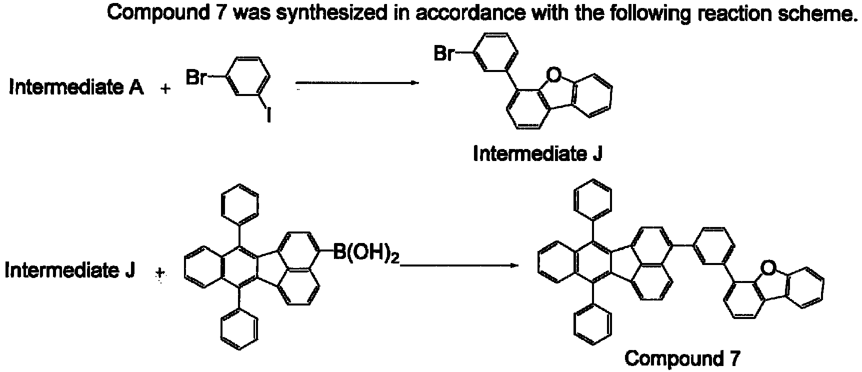

### [Synthesis of Intermediate J]

In the synthesis of Intermediate B in Example 1, the reaction was carried out in the same manner except that 1-bromo-3-iodobenzene was used in place of 6-bromo-2-naphthyl trifluoromethanesulfonate, to obtain Intermediate J.
The product was identified as Intermediate J by the FD-MS analysis.

### [Synthesis of Compound 7]

In the synthesis of Intermediate B in Example 1, the reaction was carried out in the same manner except that 7,12-diphenylbenzo[k]fluoranthen-3-ylboronic acid was used in place of Intermediate A, Intermediate J was used in place of 6-bromo-2-naphthyl trifluoromethanesulfonate, and a solvent mixture of toluene and dimethoxyethane was used in place of dimethoxyethane, to obtain Compound 7.
For the resulting Compound 7, the FDMS, and the maximum wavelength λ max of UV absorption and the maximum wavelength of fluorescence emission in a toluene solution are shown.
FDMS, calcd for C50H300=646, found m/z=646 (M+)
UV (PhMe); λmax, 420 nm, FL (PhMe, λex=375 nm); λmax, 433 nm

### Example 8

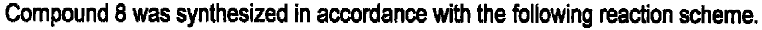

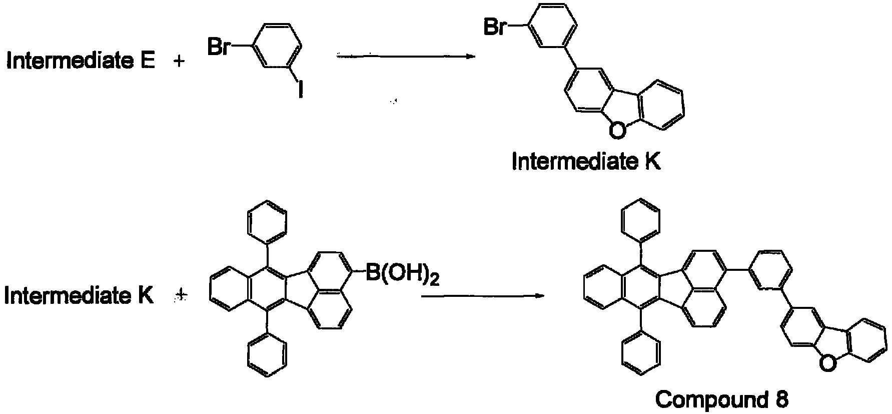

### [Synthesis of Intermediate K]

In the synthesis of Intermediate B in Example 1, the reaction was carried out in the same manner except that Intermediate E was used in place of Intermediate A, and 1-bromo-3-iodobenzene was used in place of 6-bromo-2-naphthyl trifluoromethanesulfonate, to obtain Intermediate K.
The product was identified as Intermediate K by the FD-MS analysis.

### [Synthesis of Compound 8]

In the synthesis of Intermediate B in Example 1, the reaction was carried out in the same manner except that 7,12-diphenylbenzo[k]fluoranthen-3-ylboronic acid was used in place of Intermediate A, Intermediate K was used in place of 6-bromo-2-naphthyl trifluoromethanesulfonate, and a solvent mixture of toluene and dimethoxyethane was used in place of dimethoxyethane, to obtain Compound 8.
For the resulting Compound 8, the FDMS, and the maximum wavelength λ max of UV absorption and the maximum wavelength of fluorescence emission in a toluene solution are shown.
FDMS, caled for C50H30O=646, found m/z=646 (M+)
UV (PhMe); λmax, 420 nm, FL (PhMe, λex=375 nm); λmax, 434 nm

### Example 9

A glass substrate (GEOMATEC CO., LTD.) of 25 mm by 75 mm by 1.1 mm thick with an ITO transparent electrode (anode) was subjected to ultrasonic cleaning in isopropyl alcohol for 5 minutes, and cleaned with ultraviolet rays and ozone for 30 minutes. The washed glass substrate with transparent electrode lines was mounted on a substrate holder in a vacuum deposition device. First, a film of Compound A-1 was formed by deposition, as a 60 nm-thick hole-injecting layer, on the surface of the transparent electrode on which the transparent electrode lines were formed, so as to cover the surface. Following the formation of A-1 film, on the A-1 film, as a hole-transporting layer, a 20 nm-thick film of compound A-2 was formed by deposition.
On the A-2 film, Compound H-1 of a host material and Compound 1 of a dopant material which was prepared in Example 1 were co-deposited in a film thickness ratio of 40:2 to form a 40 nm-thick film. On the film, a film of Compound A-3 was formed in a thickness of 40 nm by deposition as an electron-transporting layer Subsequently, a film of lithium fluoride was formed in a thickness of 1 nm by deposition, followed by formation of a film of aluminum in a thickness of 150 nm by deposition. The aluminum/lithium fluoride serves as a cathode. Thus, an organic EL device was fabricated.

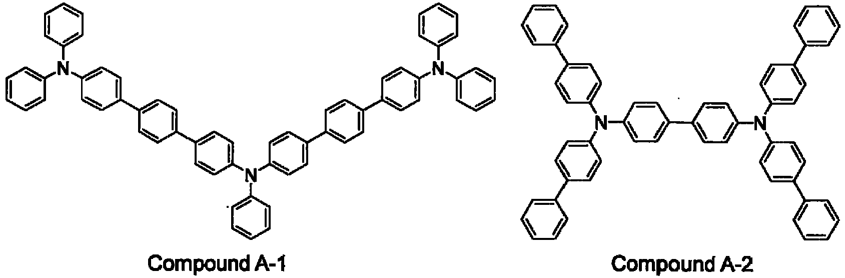

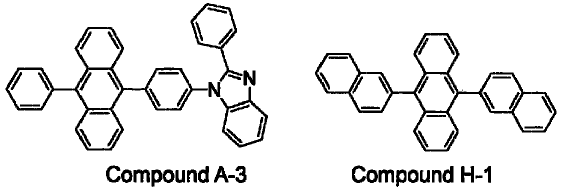

For the fabricated organic EL device, a power-on test was carried out, and the driving voltage was 3.8 V under a current density of 10 mA/cm², the luminous peak wavelength (EL λmax) was 452 nm, and the luminous efficiency was 8.2 cd/A. The device was driven in a constant current at an initial luminous intensity of 1000 cd/m², and the half-life was 6800 hours or longer. It was confirmed that the device was sufficiently practically usable. Table 1 shows the results.

### Examples 10 to 16 and Comparative Example 1

Organic EL devices were fabricated and evaluated in the same manner as in Example 9 except that compounds indicated in Table 1 were used as a dopant material in place of Compound 1, respectively. Table 1 shows the results.

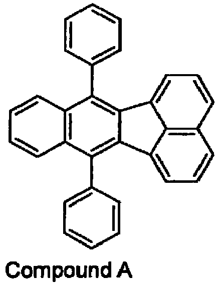

**[Table 1]**

| | Example 9 | Example 10 | Example 11 | Example 12 | Example 13 | Example 14 | Example 15 | Example 16 | Comp. Example 1 |
|---|---|---|---|---|---|---|---|---|---|
| Host material | H-1 | H-1 | H-1 | H-1 | H-1 | H-1 | H-1 | H-1 | H-1 |
| Dopant material | Compound 1 | Compound 2 | Compound 3 | Compound 4 | Compound 5 | Compound 6 | Compound 7 | Compound 8 | Compound A |
| Driving voltage (V) | 3.8 | 4.0 | 3.9 | 3.8 | 4.0 | 3.9 | 3.8 | 3.8 | 4.1 |
| EL λmax (nm) | 452 | 450 | 450 | 451 | 446 | 447 | 444 | 445 | 446 |
| Luminous efficiency (cd/A) | 8.2 | 7.8 | 7.9 | 8.0 | 7.6 | 7.6 | 7.5 | 7.5 | 2.0 |
| Lifetime (h) | 6800< | 5000< | 5500< | 6500< | 4200< | 4500< | 4000< | 4200< | 2000> |

### Industrial Applicability

Organic EL devices which use the fluoranthene compound of the invention as a material for an organic EL device, in particular, an emitting material for an organic EL device, have a high luminous efficiency and a long life.
The organic EL device of the invention is highly practical and is useful as light sources such as a plane luminous body of a wall-hanging television and a backlight of a display. The fluoranthene compound of the invention can be used as a hole-injecting or -transporting material of an organic EL device, and further as a photoconductor for an electrophotography and a charge-transporting material of an organic semiconductor.

Several embodiments and/or examples of the invention were explained above in detail. A person skilled in the art can easily add many modifications to these embodiments and/examples, without essentially deviating from the novel teachings and advantageous effects of the invention. Accordingly, these many modifications are included in the scope of the invention
The documents described in the specification are incorporated herein by reference in its entirety.

## Claims

1. A fluoranthene compound represented by the formula (1): 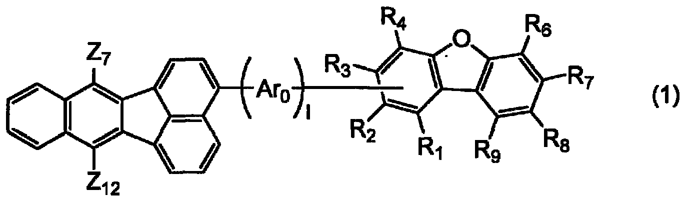 wherein
Z₇ and Z₁₂ are independently a substituted or unsubstituted aryl group having 5 to 50 ring carbon atoms, or a substituted or unsubstituted heteroaryl group having 5 to 50 ring atoms;
Ar₀ is a single bond, a substituted or unsubstituted aryl group having 5 to 50 ring carbon atoms, or a substituted or unsubstituted heteroaryl group having 5 to 50 ring atoms, and Ar₀ is a linking group bonding to any one of R₁ to R₄ and R₆ to R₉;
R₁ to R₄ and R₆ to R₉ are independently a hydrogen atom, an alkyl group, a cycloalkyl group, a substituted or unsubstituted aryl group, a substituted or unsubstituted heteroaryl group, or a substituted silyl group, or at least one pair of R₁ and R₂, R₂ and R₃, R₃ and R₄, R₆ and R₇, R₇ and R₈, and R₈ and R₉ independently bonds to each other to form a saturated or unsaturated ring structure which may have a further substituent; and
I is an integer of 1 to 4; and
when I is 2 or more, plural Ar₀s are the same or different, and substituents of adjacent Ar₀s may bond to each other.

2. The fluoranthene compound according to claim 1, wherein the fluoranthene compound is represented by the formula (2): 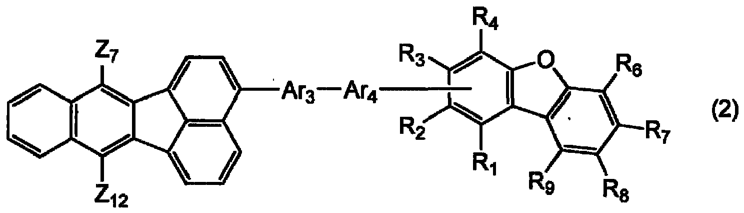 wherein
Z₇, Z₁₂, R₁ to R₄, and R₆ to Rg are the same as in the formula (1);
Ar₃ and Ar₄ are independently a substituted or unsubstituted aryl group having 5 to 50 ring carbon atoms, or a substituted or unsubstituted heteroaryl group having 5 to 50 ring atoms, and Ar₄ is a linking group bonding to any one of R₁ to R₄ and R₆ to R₉; and
a substituent of Ar₃ and a substituent of Ar₄ may bond(crosslink) to each other.

3. The fluoranthene compound according to claim 1, wherein the fluoranthene compound is represented by the formula (3): 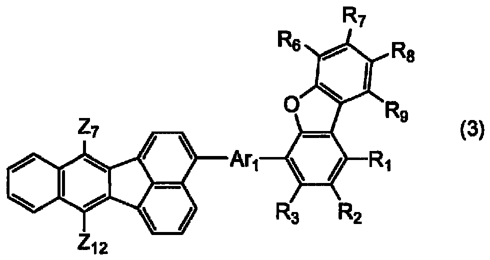 wherein
Z₇, Z₁₂, R₁ to R₃, and R₆ to R₉ are the same as in the formula (1); and
Ar₁ is a single bond, a substituted or unsubstituted aryl group having 5 to 50 ring carbon atoms, or a substituted or unsubstituted heteroaryl group having 5 to 50 ring atoms.

4. The fluoranthene compound according to claim 1, wherein the fluoranthene compound is represented by the formula (4): 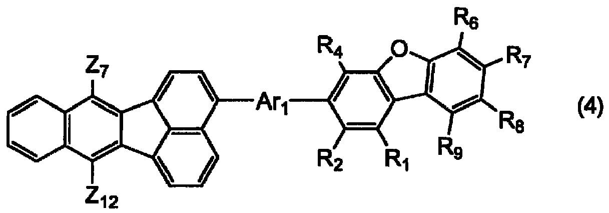 wherein
Z₇, Z₁₂, R₁, R₂, R₄, and R₆ to R₉ are the same as in the formula (1); and
Ar₁ is a single bond, a substituted or unsubstituted aryl group having 5 to 50 ring carbon atoms, or a substituted or unsubstituted heteroaryl group having 5 to 50 ring atoms.

5. The fluoranthene compound according to claim 1, wherein the fluoranthene compound is represented by the formula (5): 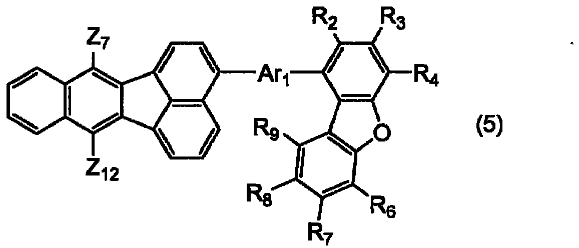 wherein
Z₇, Z₁₂, R₂ to R₄, and R₆ to R₉ are the same as in the formula (1); and
Ar₁ is a single bond, a substituted or unsubstituted aryl group having 5 to 50 ring carbon atoms, or a substituted or unsubstituted heteroaryl group having 5 to 50 ring atoms.

6. The fluoranthene compound according to claim 1, wherein the fluoranthene compound is represented by the formula (6): 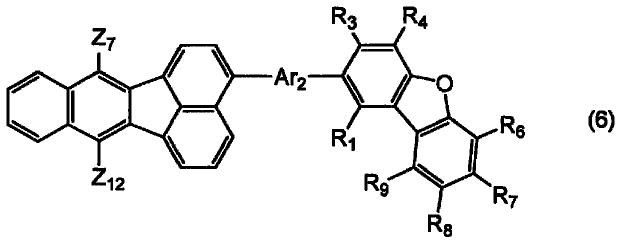 wherein
Z₇, Z₁₂, R₁, R₃, R₄, and R₆ to R₉ are the same as in the formula (1); and
Ar₂ is a single bond, a substituted or unsubstituted aryl group having 5 to 50 ring carbon atoms, or a substituted or unsubstituted heteroaryl group having 5 to 50 ring atoms.

7. The fluoranthene compound according to claim 6, wherein
Ar₂ is a single bond, a substituted or unsubstituted heteroaryl group having 5 to 50 ring atoms, or one of linking groups represented by the following formulas; and
when Ar₂ is a single bond, at least one of Tr₁, R₃, R₄, R₆, R₇, R₈ and R₉ is an alkyl group, a cycloalkyl group, a substituted or unsubstituted aryl group, a substituted or unsubstituted heteroaryl group, or a substituted silyl group. 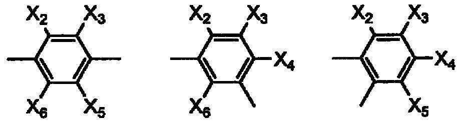 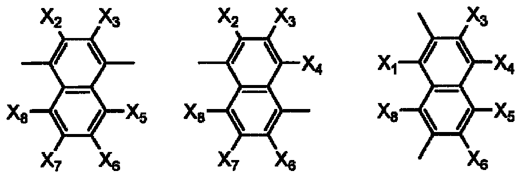 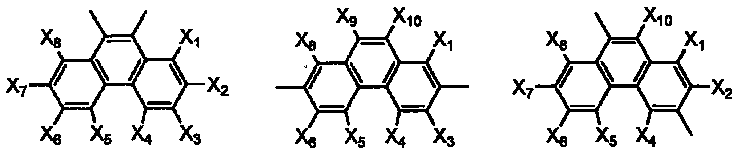 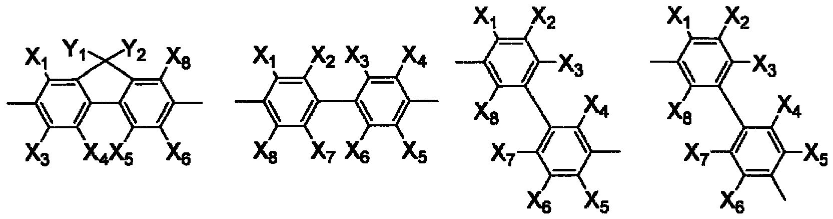 wherein
X₁ to X₁₀, Y₁ and Y₂ are independently a hydrogen atom, a fluorine atom, a cyano group, an alkyl group, a cycloalkyl group, a substituted or unsubstituted aryl group, a substituted or unsubstituted heteroaryl group, or a substituted silyl group.

8. The fluoranthene compound according to any one of claims 1 to 7, wherein
Z₇ and Z₁₂ are independently a phenyl group, a naphthyl group, a fluorenyl group, a 9,9'-dimethylfluorenyl group, a diethylfluorenyl group, a dipropylfluorenyl group, a diisopropylfluorenyl group, a dibutylfluorenyl group, a diphenylfluorenyl group, or a phenanthryl group.

9. An organic electroluminescence device which comprises:
a pair of electrodes, and
one or a plurality of organic compound layers comprising at least an emitting layer between the pair of electrodes, wherein
at least one of the organic compound layers comprises at least one of the fluoranthene compound according to any one of claims 1 to 8.

10. The organic electroluminescence device according to claim 9, wherein the emitting layer comprises the fluoranthene compound.

11. The organic electroluminescence device according to claim 10, wherein the content of the fluoranthene compound in the emitting layer is 0.01 to 20 mass %.

12. The organic electroluminescence device according to claim 10 or 11, wherein the emitting layer further comprises a compound having an anthracene central skeleton represented by the formula (2a): 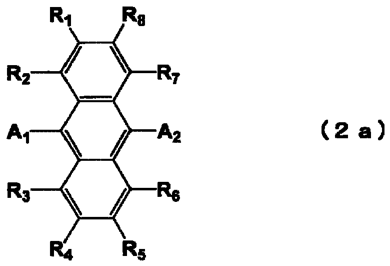 wherein
A₁ and A₂ are independently a group derived from a substituted or unsubstituted aromatic ring having 6 to 20 ring carbon atoms, and the aromatic ring may be substituted by one or two or more substituents;
the substituent is a substituted or unsubstituted aryl group having 6 to 50 ring carbon atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group, or a hydroxy group;
when the aromatic ring is substituted by two or more substituents, the substituents may be the same or different, and adjacent substituents may bond to each other to form a saturated or unsaturated ring structure; and
R₁ to R₈ are independently a hydrogen atom, a substituted or unsubstituted aryl group having 6 to 50 ring carbon atoms, a substituted or unsubstituted heteroaryl group having 5 to 50 ring atoms, a substituted or unsubstituted alkyl group having 1 to 50 carbon atoms, a substituted or unsubstituted cycloalkyl group having 3 to 50 carbon atoms, a substituted or unsubstituted alkoxy group having 1 to 50 carbon atoms, a substituted or unsubstituted aralkyl group having 6 to 50 carbon atoms, a substituted or unsubstituted aryloxy group having 5 to 50 ring atoms, a substituted or unsubstituted arylthio group having 5 to 50 ring atoms, a substituted or unsubstituted alkoxycarbonyl group having 1 to 50 carbon atoms, a substituted or unsubstituted silyl group, a carboxy group, a halogen atom, a cyano group, a nitro group, or a hydroxy group.

13. The organic electroluminescence device according to claim 12, wherein A₁ and A₂ are substituents different from each other.

14. The organic electroluminescence device according to claim 12, wherein at least one of A₁ and A₂ is a substituent having a substituted or unsubstituted fused ring group having 10 to 30 ring atoms.

15. The organic electroluminescence device according to claim 14, wherein the substituted or unsubstituted fused ring group having 10 to 30 ring atoms is a substituted or unsubstituted naphthalene ring.

16. The organic electroluminescence device according to claim 10 or 11, wherein the emitting layer further comprises a compound having a pyrene central skeleton represented by the formula (2b): 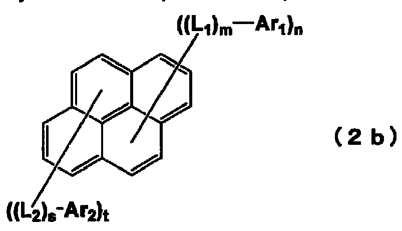 wherein
Ar₁ and Ar₂ are independently a substituted or unsubstituted aryl group having 6 to 50 ring carbon atoms;
L₁ and L₂ are independently a substituted or unsubstituted phenylene group, a substituted or unsubstituted naphthalene group, a substituted or unsubstituted fluorenylene group, or a substituted or unsubstituted dibenzosilolylene group;
m is an integer of 0 to 2, n is an integer of 1 to 4, s is an integer of 0 to 2, and t is an integer of 0 to 4; and
L₁ or Ar₁ bonds to one of the 1- to 5-positions of the pyrene, and L₂ or Ar₂ bonds to one of the 6- to 10-positions of the pyrene.

17. The organic electroluminescence device according to claim 10 or 11, wherein the emitting layer further comprises a compound having a triphenylamine skeleton represented by the formula (2c): 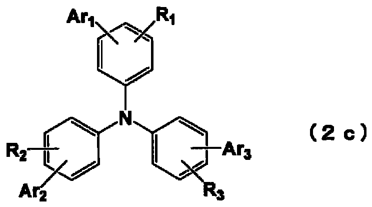 wherein
Ar₁, Ar₂ and Ar₃ are independently a group having an anthrathene structure, a group having a phenanthrene structure, or a group having a pyrene structure; and
R₁, R₂ and R₃ are independently a hydrogen atom or a substituent.

18. The organic electroluminescence device according to claim 10 or 11, wherein the emitting layer further comprises a compound represented by the formula (2d): 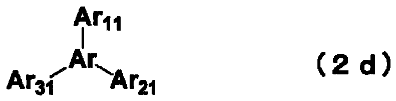 wherein
Ar₁₁, Ar₂₁ and Ar₃₁ are independently an aryl group having 6 to 50 ring carbon atoms;
the aryl group may be substituted by one or two or more substituents;
at least one of Ar₁₁, Ar₂₁ and Ar₃₁, and the substituents of these aryl groups has a fused aryl structure having 10 to 20 ring carbon atoms or a fused heteroaryl structure having 6 to 20 ring carbon atoms; and
Ar is a trivalent group derived from an aromatic ring or a heteroaromatic ring.

19. An organic electroluminescence material-containing solution which comprises:
the fluoranthene compound according to any one of claims 1 to 8 which is an organic electroluminescence material, and
a solvent.

20. The organic electroluminescence material-containing solution according to claim 19, wherein the organic electroluminescence material comprises a host material and a dopant material; the dopant material is the fluoranthene compound according to any one of claims 1 to 8; and the host material is at least one selected from the compounds represented by the formula (2a) according to claim 12, the compound represented by the formula (2b) according to claim 16, the compound represented by the formula (2c) according to claim 17, and the compound represented by the formula (2d) according to claim 18.
